# EUROPÄISCHE PATENTANMELDUNG

(11) **EP 1 767 616 A2**
(43) Veröffentlichungstag der Anmeldung: **28.03.2007**
(21) Anmeldenummer: 06119615.0
(22) Anmeldetag: 28.08.2006
(51) Int. Cl.: C12N 1/21, C12P 7/42, C12P 13/06, C12P 13/08, C12P 13/12, C12P 13/20

(54) **Verfahren zur Herstellung von L-Aminosäuren oder Ketosäuren unter Verwendung coryneformer Bakterien mit abgeschwächter aceE (pdhA) Expression**

(30) Priorität: 22.09.2005 DE 102005045301
(71) Anmelder: Degussa GmbH, 40474 Düsseldorf (DE)
(72) Erfinder: Blombach, Bastian, 86462, Stettenhofen (DE); Eikmanns, Bernhard Prof., 89077 Ulm (DE); Schreiner, Mark, 89415, Lauingen (DE); Bathe, Brigitte Dr., 33154, Salzkotten (DE); Gerstmeir, Robert Dr., 33824, Werther (DE); Thierbach, Georg Dr., 33613, Bielefeld (DE)

(57) **Zusammenfassung**

Die Erfindung betrifft ein Verfahren zur Herstellung organisch-chemischer Verbindungen unter Verwendung von coryneformen Bakterien, in denen das für die Elp Untereinheit Pyruvat-Dehydrogenase Komplexes kodierende aceE-Gen abgeschwächt vorliegt, und diese Bakterien.

SEQUENCE LISTING

<110>  Degussa AG
<120>  Verfahren zur Herstellung von organisch-chemischen Verbindungen.
<130>  050223BT
<160>  6
<170>  PatentIn version 3.3
<210>  1
<211>  2769
<212>  DNA
<213>  Corynebacterium glutamicum
<220>
<221>  CDS
<222>  (1)..(2766)
<400>  1

atg gcc gat caa gca aaa ctt ggt ggc aag ccc tcg gat gac tct aac       48
Met Ala Asp Gln Ala Lys Leu Gly Gly Lys Pro Ser Asp Asp Ser Asn
1               5                   10                  15

ttc gcg atg atc cgc gat ggc gtg gca tct tat ttg aac gac tca gat       96
Phe Ala Met Ile Arg Asp Gly Val Ala Ser Tyr Leu Asn Asp Ser Asp
            20                  25                  30

ccg gag gag acc aac gag tgg atg gat tca ctc gac gga tta ctc cag      144
Pro Glu Glu Thr Asn Glu Trp Met Asp Ser Leu Asp Gly Leu Leu Gln
        35                  40                  45

gag tct tct cca gaa cgt gct cgt tac ctc atg ctt cgt ttg ctt gag      192
Glu Ser Ser Pro Glu Arg Ala Arg Tyr Leu Met Leu Arg Leu Leu Glu
    50                  55                  60

cgt gca tct gca aag cgc gta tct ctt ccc cca atg acg tca acc gac      240
Arg Ala Ser Ala Lys Arg Val Ser Leu Pro Pro Met Thr Ser Thr Asp
65                  70                  75                  80

tac gtc aac acc att cca acc tct atg gaa cct gaa ttc cca ggc gat      288
Tyr Val Asn Thr Ile Pro Thr Ser Met Glu Pro Glu Phe Pro Gly Asp
                85                  90                  95

gag gaa atg gag aag cgt tac cgt cgt tgg att cgc tgg aac gca gcc      336
Glu Glu Met Glu Lys Arg Tyr Arg Arg Trp Ile Arg Trp Asn Ala Ala
            100                 105                 110

atc atg gtt cac cgc gct cag cga cca ggc atc ggc gtc ggc gga cac      384
Ile Met Val His Arg Ala Gln Arg Pro Gly Ile Gly Val Gly Gly His
        115                 120                 125

att tcc act tac gca ggc gca gcc cct ctg tac gaa gtt ggc ttc aac      432
Ile Ser Thr Tyr Ala Gly Ala Ala Pro Leu Tyr Glu Val Gly Phe Asn
    130                 135                 140

cac ttc ttc cgc ggc aag gat cac cca ggc ggc ggc gac cag atc ttc      480
His Phe Phe Arg Gly Lys Asp His Pro Gly Gly Gly Asp Gln Ile Phe
145                 150                 155                 160

ttc cag ggc cac gca tca cca ggt atg tac gca cgt gca ttc atg gag      528
Phe Gln Gly His Ala Ser Pro Gly Met Tyr Ala Arg Ala Phe Met Glu
                165                 170                 175

ggt cgc ctt tct gaa gac gat ctc gat ggc ttc cgt cag gaa gtt tcc 576
Gly Arg Leu Ser Glu Asp Asp Leu Asp Gly Phe Arg Gln Glu Val Ser
            180                 185                 190

cgt gag cag ggt ggc att ccg tcc tac cct cac cca cac ggt atg aag 624
Arg Glu Gln Gly Gly Ile Pro Ser Tyr Pro His Pro His Gly Met Lys
        195                 200                 205

gac ttc tgg gag ttc cca act gtg tcc atg ggt ctt ggc cca atg gat 672
Asp Phe Trp Glu Phe Pro Thr Val Ser Met Gly Leu Gly Pro Met Asp
    210                 215                 220

gcc att tac cag gca cgt ttc aac cgc tac ctc gaa aac cgt ggc atc 720
Ala Ile Tyr Gln Ala Arg Phe Asn Arg Tyr Leu Glu Asn Arg Gly Ile
225                 230                 235                 240

aag gac acc tct gac cag cac gtc tgg gcc ttc ctt ggc gac ggc gaa 768
Lys Asp Thr Ser Asp Gln His Val Trp Ala Phe Leu Gly Asp Gly Glu
                245                 250                 255

atg gac gag cca gaa tca cgt ggt ctc atc cag cag gct gca ctg aac 816
Met Asp Glu Pro Glu Ser Arg Gly Leu Ile Gln Gln Ala Ala Leu Asn
            260                 265                 270

aac ctg gac aac ctg acc ttc gtg gtt aac tgc aac ctg cag cgt ctc 864
Asn Leu Asp Asn Leu Thr Phe Val Val Asn Cys Asn Leu Gln Arg Leu
        275                 280                 285

gac gga cct gtc cgc ggt aac acc aag atc atc cag gaa ctc gag tcc 912
Asp Gly Pro Val Arg Gly Asn Thr Lys Ile Ile Gln Glu Leu Glu Ser
    290                 295                 300

ttc ttc cgt ggc gca ggc tgg tct gtg atc aag gtt gtt tgg ggt cgc 960
Phe Phe Arg Gly Ala Gly Trp Ser Val Ile Lys Val Val Trp Gly Arg
305                 310                 315                 320

gag tgg gat gaa ctt ctg gag aag gac cag gat ggt gca ctt gtt gag 1008
Glu Trp Asp Glu Leu Leu Glu Lys Asp Gln Asp Gly Ala Leu Val Glu
                325                 330                 335

atc atg aac aac acc tcc gat ggt gac tac cag acc ttc aag gct aac 1056
Ile Met Asn Asn Thr Ser Asp Gly Asp Tyr Gln Thr Phe Lys Ala Asn
            340                 345                 350

gac ggc gca tat gtt cgt gag cac ttc ttc gga cgt gac cca cgc acc 1104
Asp Gly Ala Tyr Val Arg Glu His Phe Phe Gly Arg Asp Pro Arg Thr
        355                 360                 365

gca aag ctc gtt gag aac atg acc gac gaa gaa atc tgg aag ctt cca 1152
Ala Lys Leu Val Glu Asn Met Thr Asp Glu Glu Ile Trp Lys Leu Pro
    370                 375                 380

cgt ggc ggc cac gat tac cgc aag gtt tac gca gcc tac aag cga gct 1200
Arg Gly Gly His Asp Tyr Arg Lys Val Tyr Ala Ala Tyr Lys Arg Ala
385                 390                 395                 400

ctt gag acc aag gat cgc cca acc gtc atc ctt gct cac acc att aag 1248
Leu Glu Thr Lys Asp Arg Pro Thr Val Ile Leu Ala His Thr Ile Lys
                405                 410                 415

ggc tac gga ctc ggc cac aac ttc gaa ggc cgt aac gca acc cac cag      1296
Gly Tyr Gly Leu Gly His Asn Phe Glu Gly Arg Asn Ala Thr His Gln
            420                 425                 430

atg aag aag ctg acg ctt gat gat ctg aag ttg ttc cgc gac aag cag      1344
Met Lys Lys Leu Thr Leu Asp Asp Leu Lys Leu Phe Arg Asp Lys Gln
        435                 440                 445

ggc atc cca atc acc gat gag cag ctg gag aag gat cct tac ctt cct      1392
Gly Ile Pro Ile Thr Asp Glu Gln Leu Glu Lys Asp Pro Tyr Leu Pro
    450                 455                 460

cct tac tac cac cca ggt gaa gac gct cct gaa atc aag tac atg aag      1440
Pro Tyr Tyr His Pro Gly Glu Asp Ala Pro Glu Ile Lys Tyr Met Lys
465                 470                 475                 480

gaa cgt cgc gca gcg ctc ggt ggc tac ctg cca gag cgt cgt gag aac      1488
Glu Arg Arg Ala Ala Leu Gly Gly Tyr Leu Pro Glu Arg Arg Glu Asn
                485                 490                 495

tac gat cca att cag gtt cca cca ctg gat aag ctt cgc tct gtc cgt      1536
Tyr Asp Pro Ile Gln Val Pro Pro Leu Asp Lys Leu Arg Ser Val Arg
            500                 505                 510

aag ggc tcc ggc aag cag cag atc gct acc acc atg gcg act gtt cgt      1584
Lys Gly Ser Gly Lys Gln Gln Ile Ala Thr Thr Met Ala Thr Val Arg
        515                 520                 525

acc ttc aag gaa ctg atg cgc gat aag ggc ttg gct gat cgc ctt gtc      1632
Thr Phe Lys Glu Leu Met Arg Asp Lys Gly Leu Ala Asp Arg Leu Val
    530                 535                 540

cca atc att cct gat gag gca cgt acc ttc ggt ctt gac tct tgg ttc      1680
Pro Ile Ile Pro Asp Glu Ala Arg Thr Phe Gly Leu Asp Ser Trp Phe
545                 550                 555                 560

cca acc ttg aag atc tac aac ccg cac ggt cag aac tac gtg cct gtt      1728
Pro Thr Leu Lys Ile Tyr Asn Pro His Gly Gln Asn Tyr Val Pro Val
                565                 570                 575

gac cac gac ctg atg ctc tcc tac cgt gag gca cct gaa gga cag atc      1776
Asp His Asp Leu Met Leu Ser Tyr Arg Glu Ala Pro Glu Gly Gln Ile
            580                 585                 590

ctg cac gaa ggc atc aac gag gct ggt tcc gtg gca tcg ttc atc gct      1824
Leu His Glu Gly Ile Asn Glu Ala Gly Ser Val Ala Ser Phe Ile Ala
        595                 600                 605

gcg ggt acc tcc tac gcc acc cac ggc aag gcc atg att ccg ctg tac      1872
Ala Gly Thr Ser Tyr Ala Thr His Gly Lys Ala Met Ile Pro Leu Tyr
    610                 615                 620

atc ttc tac tcg atg ttc gga ttc cag cgc acc ggt gac tcc atc tgg      1920
Ile Phe Tyr Ser Met Phe Gly Phe Gln Arg Thr Gly Asp Ser Ile Trp
625                 630                 635                 640

gca gca gcc gat cag atg gca cgt ggc ttc ctc ttg ggc gct acc gca      1968
Ala Ala Ala Asp Gln Met Ala Arg Gly Phe Leu Leu Gly Ala Thr Ala
                645                 650                 655

ggt cgc acc acc ctg acc ggt gaa ggc ctc cag cac atg gat gga cac      2016
Gly Arg Thr Thr Leu Thr Gly Glu Gly Leu Gln His Met Asp Gly His
            660                 665                 670

tcc cct gtc ttg gct tcc acc aac gag ggt gtc gag acc tac gac cca      2064
Ser Pro Val Leu Ala Ser Thr Asn Glu Gly Val Glu Thr Tyr Asp Pro
        675                 680                 685

tcc ttt gcg tac gag atc gca cac ctg gtt cac cgt ggc atc gac cgc      2112
Ser Phe Ala Tyr Glu Ile Ala His Leu Val His Arg Gly Ile Asp Arg
    690                 695                 700

atg tac ggc cca ggc aag ggt gaa gat gtt atc tac tac atc acc atc      2160
Met Tyr Gly Pro Gly Lys Gly Glu Asp Val Ile Tyr Tyr Ile Thr Ile
705                 710                 715                 720

tac aac gag cca acc cca cag cca gct gag cca gaa gga ctg gac gta      2208
Tyr Asn Glu Pro Thr Pro Gln Pro Ala Glu Pro Glu Gly Leu Asp Val
                725                 730                 735

gaa ggc ctg cac aag ggc atc tac ctc tac tcc cgc ggt gaa ggc acc      2256
Glu Gly Leu His Lys Gly Ile Tyr Leu Tyr Ser Arg Gly Glu Gly Thr
            740                 745                 750

ggc cat gag gca aac atc ttg gct tcc ggt gtt ggt atg cag tgg gct      2304
Gly His Glu Ala Asn Ile Leu Ala Ser Gly Val Gly Met Gln Trp Ala
        755                 760                 765

ctc aag gct gca tcc atc ctt gag gct gac tac gga gtt cgt gcc aac      2352
Leu Lys Ala Ala Ser Ile Leu Glu Ala Asp Tyr Gly Val Arg Ala Asn
    770                 775                 780

att tac tcc gct act tct tgg gtt aac ttg gct cgc gat ggc gct gct      2400
Ile Tyr Ser Ala Thr Ser Trp Val Asn Leu Ala Arg Asp Gly Ala Ala
785                 790                 795                 800

cgt aac aag gca cag ctg cgc aac cca ggt gca gat gct ggc gag gca      2448
Arg Asn Lys Ala Gln Leu Arg Asn Pro Gly Ala Asp Ala Gly Glu Ala
                805                 810                 815

ttc gta acc acc cag ctg aag cag acc tcc ggc cca tac gtt gca gtg      2496
Phe Val Thr Thr Gln Leu Lys Gln Thr Ser Gly Pro Tyr Val Ala Val
            820                 825                 830

tct gac ttc tcc act gat ctg cca aac cag atc cgt gaa tgg gtc cca      2544
Ser Asp Phe Ser Thr Asp Leu Pro Asn Gln Ile Arg Glu Trp Val Pro
        835                 840                 845

ggc gac tac acc gtt ctc ggt gca gat ggc ttc ggt ttc tct gat acc      2592
Gly Asp Tyr Thr Val Leu Gly Ala Asp Gly Phe Gly Phe Ser Asp Thr
    850                 855                 860

cgc cca gct gct cgt cgc ttc ttc aac atc gac gct gag tcc att gtt      2640
Arg Pro Ala Ala Arg Arg Phe Phe Asn Ile Asp Ala Glu Ser Ile Val
865                 870                 875                 880

gtt gca gtg ctg aac tcc ctg gca cgc gaa ggc aag atc gac gtc tcc      2688
Val Ala Val Leu Asn Ser Leu Ala Arg Glu Gly Lys Ile Asp Val Ser
                885                 890                 895

gtt gct gct cag gct gct gag aag ttc aag ttg gat gat cct acg agt     2736
Val Ala Ala Gln Ala Ala Glu Lys Phe Lys Leu Asp Asp Pro Thr Ser
            900                 905                 910

gtt tcc gta gat cca aac gct cct gag gaa taa                         2769
Val Ser Val Asp Pro Asn Ala Pro Glu Glu
        915                 920

<210>  2
<211>  922
<212>  PRT
<213>  Corynebacterium glutamicum

<400>  2

Met Ala Asp Gln Ala Lys Leu Gly Gly Lys Pro Ser Asp Asp Ser Asn
1               5                   10                  15

Phe Ala Met Ile Arg Asp Gly Val Ala Ser Tyr Leu Asn Asp Ser Asp
            20                  25                  30

Pro Glu Glu Thr Asn Glu Trp Met Asp Ser Leu Asp Gly Leu Leu Gln
        35                  40                  45

Glu Ser Ser Pro Glu Arg Ala Arg Tyr Leu Met Leu Arg Leu Leu Glu
    50                  55                  60

Arg Ala Ser Ala Lys Arg Val Ser Leu Pro Pro Met Thr Ser Thr Asp
65                  70                  75                  80

Tyr Val Asn Thr Ile Pro Thr Ser Met Glu Pro Glu Phe Pro Gly Asp
                85                  90                  95

Glu Glu Met Glu Lys Arg Tyr Arg Arg Trp Ile Arg Trp Asn Ala Ala
            100                 105                 110

Ile Met Val His Arg Ala Gln Arg Pro Gly Ile Gly Val Gly Gly His
        115                 120                 125

Ile Ser Thr Tyr Ala Gly Ala Ala Pro Leu Tyr Glu Val Gly Phe Asn
    130                 135                 140

His Phe Phe Arg Gly Lys Asp His Pro Gly Gly Gly Asp Gln Ile Phe
145                 150                 155                 160

Phe Gln Gly His Ala Ser Pro Gly Met Tyr Ala Arg Ala Phe Met Glu
                165                 170                 175

Gly Arg Leu Ser Glu Asp Asp Leu Asp Gly Phe Arg Gln Glu Val Ser
            180                 185                 190

Arg Glu Gln Gly Gly Ile Pro Ser Tyr Pro His Pro His Gly Met Lys
        195                 200                 205

Asp Phe Trp Glu Phe Pro Thr Val Ser Met Gly Leu Gly Pro Met Asp
    210                 215                 220

Ala Ile Tyr Gln Ala Arg Phe Asn Arg Tyr Leu Glu Asn Arg Gly Ile
225                 230                 235                 240

Lys Asp Thr Ser Asp Gln His Val Trp Ala Phe Leu Gly Asp Gly Glu
                245                 250                 255

Met Asp Glu Pro Glu Ser Arg Gly Leu Ile Gln Gln Ala Ala Leu Asn
            260                 265                 270

Asn Leu Asp Asn Leu Thr Phe Val Val Asn Cys Asn Leu Gln Arg Leu
        275                 280                 285

Asp Gly Pro Val Arg Gly Asn Thr Lys Ile Ile Gln Glu Leu Glu Ser
    290                 295                 300

Phe Phe Arg Gly Ala Gly Trp Ser Val Ile Lys Val Val Trp Gly Arg
305                 310                 315                 320

Glu Trp Asp Glu Leu Leu Glu Lys Asp Gln Asp Gly Ala Leu Val Glu
                325                 330                 335

Ile Met Asn Asn Thr Ser Asp Gly Asp Tyr Gln Thr Phe Lys Ala Asn
            340                 345                 350

Asp Gly Ala Tyr Val Arg Glu His Phe Phe Gly Arg Asp Pro Arg Thr
        355                 360                 365

Ala Lys Leu Val Glu Asn Met Thr Asp Glu Glu Ile Trp Lys Leu Pro
    370                 375                 380

Arg Gly Gly His Asp Tyr Arg Lys Val Tyr Ala Ala Tyr Lys Arg Ala
385                 390                 395                 400

Leu Glu Thr Lys Asp Arg Pro Thr Val Ile Leu Ala His Thr Ile Lys
                405                 410                 415

Gly Tyr Gly Leu Gly His Asn Phe Glu Gly Arg Asn Ala Thr His Gln
            420                 425                 430

Met Lys Lys Leu Thr Leu Asp Asp Leu Lys Leu Phe Arg Asp Lys Gln
        435                 440                 445

Gly Ile Pro Ile Thr Asp Glu Gln Leu Glu Lys Asp Pro Tyr Leu Pro
    450                 455                 460

Pro Tyr Tyr His Pro Gly Glu Asp Ala Pro Glu Ile Lys Tyr Met Lys
465                 470                 475                 480

Glu Arg Arg Ala Ala Leu Gly Gly Tyr Leu Pro Glu Arg Arg Glu Asn
                485                 490                 495

Tyr Asp Pro Ile Gln Val Pro Pro Leu Asp Lys Leu Arg Ser Val Arg
            500                 505                 510

Lys Gly Ser Gly Lys Gln Gln Ile Ala Thr Thr Met Ala Thr Val Arg
        515                 520                 525

Thr Phe Lys Glu Leu Met Arg Asp Lys Gly Leu Ala Asp Arg Leu Val
    530                 535                 540

Pro Ile Ile Pro Asp Glu Ala Arg Thr Phe Gly Leu Asp Ser Trp Phe
545                 550                 555                 560

Pro Thr Leu Lys Ile Tyr Asn Pro His Gly Gln Asn Tyr Val Pro Val
                565                 570                 575

Asp His Asp Leu Met Leu Ser Tyr Arg Glu Ala Pro Glu Gly Gln Ile
            580                 585                 590

Leu His Glu Gly Ile Asn Glu Ala Gly Ser Val Ala Ser Phe Ile Ala
        595                 600                 605

Ala Gly Thr Ser Tyr Ala Thr His Gly Lys Ala Met Ile Pro Leu Tyr
    610                 615                 620

Ile Phe Tyr Ser Met Phe Gly Phe Gln Arg Thr Gly Asp Ser Ile Trp
625                 630                 635                 640

Ala Ala Ala Asp Gln Met Ala Arg Gly Phe Leu Leu Gly Ala Thr Ala
                645                 650                 655

Gly Arg Thr Thr Leu Thr Gly Glu Gly Leu Gln His Met Asp Gly His
            660                 665                 670

Ser Pro Val Leu Ala Ser Thr Asn Glu Gly Val Glu Thr Tyr Asp Pro
        675                 680                 685

Ser Phe Ala Tyr Glu Ile Ala His Leu Val His Arg Gly Ile Asp Arg
    690                 695                 700

Met Tyr Gly Pro Gly Lys Gly Glu Asp Val Ile Tyr Tyr Ile Thr Ile
705                 710                 715                 720

Tyr Asn Glu Pro Thr Pro Gln Pro Ala Glu Pro Glu Gly Leu Asp Val
                725                 730                 735

Glu Gly Leu His Lys Gly Ile Tyr Leu Tyr Ser Arg Gly Glu Gly Thr
            740                 745                 750

Gly His Glu Ala Asn Ile Leu Ala Ser Gly Val Gly Met Gln Trp Ala
        755                 760                 765

Leu Lys Ala Ala Ser Ile Leu Glu Ala Asp Tyr Gly Val Arg Ala Asn
    770                 775                 780

Ile Tyr Ser Ala Thr Ser Trp Val Asn Leu Ala Arg Asp Gly Ala Ala
785                 790                 795                 800

Arg Asn Lys Ala Gln Leu Arg Asn Pro Gly Ala Asp Ala Gly Glu Ala
                805                 810                 815

Phe Val Thr Thr Gln Leu Lys Gln Thr Ser Gly Pro Tyr Val Ala Val
            820                 825                 830

Ser Asp Phe Ser Thr Asp Leu Pro Asn Gln Ile Arg Glu Trp Val Pro
        835                 840                 845

Gly Asp Tyr Thr Val Leu Gly Ala Asp Gly Phe Gly Phe Ser Asp Thr
    850                 855                 860

Arg Pro Ala Ala Arg Arg Phe Phe Asn Ile Asp Ala Glu Ser Ile Val
865                 870                 875                 880

Val Ala Val Leu Asn Ser Leu Ala Arg Glu Gly Lys Ile Asp Val Ser
                885                 890                 895

Val Ala Ala Gln Ala Ala Glu Lys Phe Lys Leu Asp Asp Pro Thr Ser
            900                 905                 910

Val Ser Val Asp Pro Asn Ala Pro Glu Glu
        915                 920

<210>  3
<211>  3769
<212>  DNA
<213>  Corynebacterium glutamicum

<220>
<221>  CDS
<222>  (501)..(3266)

<400>  3
aatcaatgaa ttcaatcact ggccagcgat taacttttcg agttttcagt cttggatttc      60

cacaattctc ttcaaaataa tggtggctag atttttcatc aaaccctcac caaaaggaca     120

tcagacctgt agttttatgc gattcgcgtc aaacgtgaga gaaacatcac atctcgcggg     180

aaactacccg ataattcttt gcaaaacttt gcaaagcgga atgaacatgc agctagtttc     240

cgtagaaatg ttctttaaaa aatccacaac aattgccagg aagcacaccg attgatggat     300

acctgaaatc ccagtgagcg caccgctccc cttacgtcac agtctgtaaa acaaatcttc     360

ggtgttgcgt atccttgtta ataacttatg cgttgactca ttcgtgcact tcggcgtgtc     420

acaattaggt acgaccaaga atgggaccgg gaaaccggga cgtataaacg aaataaaaca     480

ttccaacagg aggtgtggaa atg gcc gat caa gca aaa ctt ggt ggc aag ccc     533
                      Met Ala Asp Gln Ala Lys Leu Gly Gly Lys Pro
                      1               5                   10

tcg gat gac tct aac ttc gcg atg atc cgc gat ggc gtg gca tct tat       581
Ser Asp Asp Ser Asn Phe Ala Met Ile Arg Asp Gly Val Ala Ser Tyr
            15                  20                  25

ttg aac gac tca gat ccg gag gag acc aac gag tgg atg gat tca ctc       629
Leu Asn Asp Ser Asp Pro Glu Glu Thr Asn Glu Trp Met Asp Ser Leu
        30                  35                  40

gac gga tta ctc cag gag tct tct cca gaa cgt gct cgt tac ctc atg       677
Asp Gly Leu Leu Gln Glu Ser Ser Pro Glu Arg Ala Arg Tyr Leu Met
    45                  50                  55

ctt cgt ttg ctt gag cgt gca tct gca aag cgc gta tct ctt ccc cca       725
Leu Arg Leu Leu Glu Arg Ala Ser Ala Lys Arg Val Ser Leu Pro Pro
60                  65                  70                  75

atg acg tca acc gac tac gtc aac acc att cca acc tct atg gaa cct       773
Met Thr Ser Thr Asp Tyr Val Asn Thr Ile Pro Thr Ser Met Glu Pro
                80                  85                  90

gaa ttc cca ggc gat gag gaa atg gag aag cgt tac cgt cgt tgg att       821
Glu Phe Pro Gly Asp Glu Glu Met Glu Lys Arg Tyr Arg Arg Trp Ile
            95                  100                 105

cgc tgg aac gca gcc atc atg gtt cac cgc gct cag cga cca ggc atc       869
Arg Trp Asn Ala Ala Ile Met Val His Arg Ala Gln Arg Pro Gly Ile
        110                 115                 120

ggc gtc ggc gga cac att tcc act tac gca ggc gca gcc cct ctg tac     917
Gly Val Gly Gly His Ile Ser Thr Tyr Ala Gly Ala Ala Pro Leu Tyr
    125                 130                 135

gaa gtt ggc ttc aac cac ttc ttc cgc ggc aag gat cac cca ggc ggc     965
Glu Val Gly Phe Asn His Phe Phe Arg Gly Lys Asp His Pro Gly Gly
140                 145                 150                 155

ggc gac cag atc ttc ttc cag ggc cac gca tca cca ggt atg tac gca     1013
Gly Asp Gln Ile Phe Phe Gln Gly His Ala Ser Pro Gly Met Tyr Ala
                160                 165                 170

cgt gca ttc atg gag ggt cgc ctt tct gaa gac gat ctc gat ggc ttc     1061
Arg Ala Phe Met Glu Gly Arg Leu Ser Glu Asp Asp Leu Asp Gly Phe
            175                 180                 185

cgt cag gaa gtt tcc cgt gag cag ggt ggc att ccg tcc tac cct cac     1109
Arg Gln Glu Val Ser Arg Glu Gln Gly Gly Ile Pro Ser Tyr Pro His
        190                 195                 200

cca cac ggt atg aag gac ttc tgg gag ttc cca act gtg tcc atg ggt     1157
Pro His Gly Met Lys Asp Phe Trp Glu Phe Pro Thr Val Ser Met Gly
    205                 210                 215

ctt ggc cca atg gat gcc att tac cag gca cgt ttc aac cgc tac ctc     1205
Leu Gly Pro Met Asp Ala Ile Tyr Gln Ala Arg Phe Asn Arg Tyr Leu
220                 225                 230                 235

gaa aac cgt ggc atc aag gac acc tct gac cag cac gtc tgg gcc ttc     1253
Glu Asn Arg Gly Ile Lys Asp Thr Ser Asp Gln His Val Trp Ala Phe
                240                 245                 250

ctt ggc gac ggc gaa atg gac gag cca gaa tca cgt ggt ctc atc cag     1301
Leu Gly Asp Gly Glu Met Asp Glu Pro Glu Ser Arg Gly Leu Ile Gln
            255                 260                 265

cag gct gca ctg aac aac ctg gac aac ctg acc ttc gtg gtt aac tgc     1349
Gln Ala Ala Leu Asn Asn Leu Asp Asn Leu Thr Phe Val Val Asn Cys
        270                 275                 280

aac ctg cag cgt ctc gac gga cct gtc cgc ggt aac acc aag atc atc     1397
Asn Leu Gln Arg Leu Asp Gly Pro Val Arg Gly Asn Thr Lys Ile Ile
    285                 290                 295

cag gaa ctc gag tcc ttc ttc cgt ggc gca ggc tgg tct gtg atc aag     1445
Gln Glu Leu Glu Ser Phe Phe Arg Gly Ala Gly Trp Ser Val Ile Lys
300                 305                 310                 315

gtt gtt tgg ggt cgc gag tgg gat gaa ctt ctg gag aag gac cag gat     1493
Val Val Trp Gly Arg Glu Trp Asp Glu Leu Leu Glu Lys Asp Gln Asp
                320                 325                 330

ggt gca ctt gtt gag atc atg aac aac acc tcc gat ggt gac tac cag     1541
Gly Ala Leu Val Glu Ile Met Asn Asn Thr Ser Asp Gly Asp Tyr Gln
            335                 340                 345

acc ttc aag gct aac gac ggc gca tat gtt cgt gag cac ttc ttc gga     1589
Thr Phe Lys Ala Asn Asp Gly Ala Tyr Val Arg Glu His Phe Phe Gly
        350                 355                 360

cgt gac cca cgc acc gca aag ctc gtt gag aac atg acc gac gaa gaa     1637
Arg Asp Pro Arg Thr Ala Lys Leu Val Glu Asn Met Thr Asp Glu Glu
    365                 370                 375

atc tgg aag ctt cca cgt ggc ggc cac gat tac cgc aag gtt tac gca     1685
Ile Trp Lys Leu Pro Arg Gly Gly His Asp Tyr Arg Lys Val Tyr Ala
380                 385                 390                 395

gcc tac aag cga gct ctt gag acc aag gat cgc cca acc gtc atc ctt     1733
Ala Tyr Lys Arg Ala Leu Glu Thr Lys Asp Arg Pro Thr Val Ile Leu
                400                 405                 410

gct cac acc att aag ggc tac gga ctc ggc cac aac ttc gaa ggc cgt     1781
Ala His Thr Ile Lys Gly Tyr Gly Leu Gly His Asn Phe Glu Gly Arg
            415                 420                 425

aac gca acc cac cag atg aag aag ctg acg ctt gat gat ctg aag ttg     1829
Asn Ala Thr His Gln Met Lys Lys Leu Thr Leu Asp Asp Leu Lys Leu
        430                 435                 440

ttc cgc gac aag cag ggc atc cca atc acc gat gag cag ctg gag aag     1877
Phe Arg Asp Lys Gln Gly Ile Pro Ile Thr Asp Glu Gln Leu Glu Lys
    445                 450                 455

gat cct tac ctt cct cct tac tac cac cca ggt gaa gac gct cct gaa     1925
Asp Pro Tyr Leu Pro Pro Tyr Tyr His Pro Gly Glu Asp Ala Pro Glu
460                 465                 470                 475

atc aag tac atg aag gaa cgt cgc gca gcg ctc ggt ggc tac ctg cca     1973
Ile Lys Tyr Met Lys Glu Arg Arg Ala Ala Leu Gly Gly Tyr Leu Pro
                480                 485                 490

gag cgt cgt gag aac tac gat cca att cag gtt cca cca ctg gat aag     2021
Glu Arg Arg Glu Asn Tyr Asp Pro Ile Gln Val Pro Pro Leu Asp Lys
            495                 500                 505

ctt cgc tct gtc cgt aag ggc tcc ggc aag cag cag atc gct acc acc     2069
Leu Arg Ser Val Arg Lys Gly Ser Gly Lys Gln Gln Ile Ala Thr Thr
        510                 515                 520

atg gcg act gtt cgt acc ttc aag gaa ctg atg cgc gat aag ggc ttg     2117
Met Ala Thr Val Arg Thr Phe Lys Glu Leu Met Arg Asp Lys Gly Leu
    525                 530                 535

gct gat cgc ctt gtc cca atc att cct gat gag gca cgt acc ttc ggt     2165
Ala Asp Arg Leu Val Pro Ile Ile Pro Asp Glu Ala Arg Thr Phe Gly
540                 545                 550                 555

ctt gac tct tgg ttc cca acc ttg aag atc tac aac ccg cac ggt cag     2213
Leu Asp Ser Trp Phe Pro Thr Leu Lys Ile Tyr Asn Pro His Gly Gln
                560                 565                 570

aac tac gtg cct gtt gac cac gac ctg atg ctc tcc tac cgt gag gca     2261
Asn Tyr Val Pro Val Asp His Asp Leu Met Leu Ser Tyr Arg Glu Ala
            575                 580                 585

cct gaa gga cag atc ctg cac gaa ggc atc aac gag gct ggt tcc gtg     2309
Pro Glu Gly Gln Ile Leu His Glu Gly Ile Asn Glu Ala Gly Ser Val
        590                 595                 600

gca tcg ttc atc gct gcg ggt acc tcc tac gcc acc cac ggc aag gcc     2357
Ala Ser Phe Ile Ala Ala Gly Thr Ser Tyr Ala Thr His Gly Lys Ala
    605                 610                 615

atg att ccg ctg tac atc ttc tac tcg atg ttc gga ttc cag cgc acc     2405
Met Ile Pro Leu Tyr Ile Phe Tyr Ser Met Phe Gly Phe Gln Arg Thr
620                 625                 630                 635

ggt gac tcc atc tgg gca gca gcc gat cag atg gca cgt ggc ttc ctc     2453
Gly Asp Ser Ile Trp Ala Ala Ala Asp Gln Met Ala Arg Gly Phe Leu
                640                 645                 650

ttg ggc gct acc gca ggt cgc acc acc ctg acc ggt gaa ggc ctc cag     2501
Leu Gly Ala Thr Ala Gly Arg Thr Thr Leu Thr Gly Glu Gly Leu Gln
            655                 660                 665

cac atg gat gga cac tcc cct gtc ttg gct tcc acc aac gag ggt gtc     2549
His Met Asp Gly His Ser Pro Val Leu Ala Ser Thr Asn Glu Gly Val
        670                 675                 680

gag acc tac gac cca tcc ttt gcg tac gag atc gca cac ctg gtt cac     2597
Glu Thr Tyr Asp Pro Ser Phe Ala Tyr Glu Ile Ala His Leu Val His
    685                 690                 695

cgt ggc atc gac cgc atg tac ggc cca ggc aag ggt gaa gat gtt atc     2645
Arg Gly Ile Asp Arg Met Tyr Gly Pro Gly Lys Gly Glu Asp Val Ile
700                 705                 710                 715

tac tac atc acc atc tac aac gag cca acc cca cag cca gct gag cca     2693
Tyr Tyr Ile Thr Ile Tyr Asn Glu Pro Thr Pro Gln Pro Ala Glu Pro
                720                 725                 730

gaa gga ctg gac gta gaa ggc ctg cac aag ggc atc tac ctc tac tcc     2741
Glu Gly Leu Asp Val Glu Gly Leu His Lys Gly Ile Tyr Leu Tyr Ser
            735                 740                 745

cgc ggt gaa ggc acc ggc cat gag gca aac atc ttg gct tcc ggt gtt     2789
Arg Gly Glu Gly Thr Gly His Glu Ala Asn Ile Leu Ala Ser Gly Val
        750                 755                 760

ggt atg cag tgg gct ctc aag gct gca tcc atc ctt gag gct gac tac     2837
Gly Met Gln Trp Ala Leu Lys Ala Ala Ser Ile Leu Glu Ala Asp Tyr
    765                 770                 775

gga gtt cgt gcc aac att tac tcc gct act tct tgg gtt aac ttg gct     2885
Gly Val Arg Ala Asn Ile Tyr Ser Ala Thr Ser Trp Val Asn Leu Ala
780                 785                 790                 795

cgc gat ggc gct gct cgt aac aag gca cag ctg cgc aac cca ggt gca     2933
Arg Asp Gly Ala Ala Arg Asn Lys Ala Gln Leu Arg Asn Pro Gly Ala
                800                 805                 810

gat gct ggc gag gca ttc gta acc acc cag ctg aag cag acc tcc ggc     2981
Asp Ala Gly Glu Ala Phe Val Thr Thr Gln Leu Lys Gln Thr Ser Gly
            815                 820                 825

cca tac gtt gca gtg tct gac ttc tcc act gat ctg cca aac cag atc     3029
Pro Tyr Val Ala Val Ser Asp Phe Ser Thr Asp Leu Pro Asn Gln Ile
        830                 835                 840

cgt gaa tgg gtc cca ggc gac tac acc gtt ctc ggt gca gat ggc ttc     3077
Arg Glu Trp Val Pro Gly Asp Tyr Thr Val Leu Gly Ala Asp Gly Phe
    845                 850                 855

ggt ttc tct gat acc cgc cca gct gct cgt cgc ttc ttc aac atc gac     3125
Gly Phe Ser Asp Thr Arg Pro Ala Ala Arg Arg Phe Phe Asn Ile Asp
860                 865                 870                 875

gct gag tcc att gtt gtt gca gtg ctg aac tcc ctg gca cgc gaa ggc     3173
Ala Glu Ser Ile Val Val Ala Val Leu Asn Ser Leu Ala Arg Glu Gly
                880                 885                 890

aag atc gac gtc tcc gtt gct gct cag gct gct gag aag ttc aag ttg     3221
Lys Ile Asp Val Ser Val Ala Ala Gln Ala Ala Glu Lys Phe Lys Leu
            895                 900                 905

gat gat cct acg agt gtt tcc gta gat cca aac gct cct gag gaa         3266
Asp Asp Pro Thr Ser Val Ser Val Asp Pro Asn Ala Pro Glu Glu
        910                 915                 920

taaatcacct caagggacag ataaatcccg ccgccagacg ttagtctggc ggcggcatcc   3326

gtcgtaaagc aagctctttt tagccgagga acgccttgtc agacaatgtt gcgcccttga   3386

tgttggcgaa ctcctgcagc aaatcgcgca cagtcaactt cgacttggta gcctgatctg   3446

cctggtagac aatctggcct tcatgcatca tgatcacgcg attgcccagg cgaattgcct   3506

gttccatgtt gtgcgtgacc ataagcgtag tcagatttcc atctgccacg atcttttcgg   3566

tcaaggtggt cacaagctct gcacgctgtg gatcaagcgc tgcggtgtgc tcatccaaca   3626

gcatgatttt aggttgagta aaaccagcca tcagcaggga caatgcctga cgctgaccgc   3686

cagagagcaa accaactttg gcagtgagcc tgttttccag acccagctca aggcgctcaa   3746

gttcctgctt gaattgctca cgg                                           3769

<210>  4
<211>  922
<212>  PRT
<213>  Corynebacterium glutamicum

<400>  4

Met Ala Asp Gln Ala Lys Leu Gly Gly Lys Pro Ser Asp Asp Ser Asn
1               5                   10                  15

Phe Ala Met Ile Arg Asp Gly Val Ala Ser Tyr Leu Asn Asp Ser Asp
            20                  25                  30

Pro Glu Glu Thr Asn Glu Trp Met Asp Ser Leu Asp Gly Leu Leu Gln
        35                  40                  45

Glu Ser Ser Pro Glu Arg Ala Arg Tyr Leu Met Leu Arg Leu Leu Glu
    50                  55                  60

Arg Ala Ser Ala Lys Arg Val Ser Leu Pro Pro Met Thr Ser Thr Asp
65                  70                  75                  80

Tyr Val Asn Thr Ile Pro Thr Ser Met Glu Pro Glu Phe Pro Gly Asp
                85                  90                  95

Glu Glu Met Glu Lys Arg Tyr Arg Arg Trp Ile Arg Trp Asn Ala Ala
            100                 105                 110

Ile Met Val His Arg Ala Gln Arg Pro Gly Ile Gly Val Gly Gly His
        115                 120                 125

Ile Ser Thr Tyr Ala Gly Ala Ala Pro Leu Tyr Glu Val Gly Phe Asn
    130                 135                 140

His Phe Phe Arg Gly Lys Asp His Pro Gly Gly Gly Asp Gln Ile Phe
145                 150                 155                 160

Phe Gln Gly His Ala Ser Pro Gly Met Tyr Ala Arg Ala Phe Met Glu
                165                 170                 175

Gly Arg Leu Ser Glu Asp Asp Leu Asp Gly Phe Arg Gln Glu Val Ser
            180                 185                 190

Arg Glu Gln Gly Gly Ile Pro Ser Tyr Pro His Pro His Gly Met Lys
        195                 200                 205

Asp Phe Trp Glu Phe Pro Thr Val Ser Met Gly Leu Gly Pro Met Asp
    210                 215                 220

Ala Ile Tyr Gln Ala Arg Phe Asn Arg Tyr Leu Glu Asn Arg Gly Ile
225                 230                 235                 240

Lys Asp Thr Ser Asp Gln His Val Trp Ala Phe Leu Gly Asp Gly Glu
                245                 250                 255

Met Asp Glu Pro Glu Ser Arg Gly Leu Ile Gln Gln Ala Ala Leu Asn
            260                 265                 270

Asn Leu Asp Asn Leu Thr Phe Val Val Asn Cys Asn Leu Gln Arg Leu
        275                 280                 285

Asp Gly Pro Val Arg Gly Asn Thr Lys Ile Ile Gln Glu Leu Glu Ser
    290                 295                 300

Phe Phe Arg Gly Ala Gly Trp Ser Val Ile Lys Val Val Trp Gly Arg
305                 310                 315                 320

Glu Trp Asp Glu Leu Leu Glu Lys Asp Gln Asp Gly Ala Leu Val Glu
                325                 330                 335

Ile Met Asn Asn Thr Ser Asp Gly Asp Tyr Gln Thr Phe Lys Ala Asn
            340                 345                 350

Asp Gly Ala Tyr Val Arg Glu His Phe Phe Gly Arg Asp Pro Arg Thr
        355                 360                 365

Ala Lys Leu Val Glu Asn Met Thr Asp Glu Glu Ile Trp Lys Leu Pro
    370                 375                 380

Arg Gly Gly His Asp Tyr Arg Lys Val Tyr Ala Ala Tyr Lys Arg Ala
385                 390                 395                 400

Leu Glu Thr Lys Asp Arg Pro Thr Val Ile Leu Ala His Thr Ile Lys
                405                 410                 415

Gly Tyr Gly Leu Gly His Asn Phe Glu Gly Arg Asn Ala Thr His Gln
            420                 425                 430

Met Lys Lys Leu Thr Leu Asp Asp Leu Lys Leu Phe Arg Asp Lys Gln
        435                 440                 445

Gly Ile Pro Ile Thr Asp Glu Gln Leu Glu Lys Asp Pro Tyr Leu Pro
    450                 455                 460

Pro Tyr Tyr His Pro Gly Glu Asp Ala Pro Glu Ile Lys Tyr Met Lys
465                 470                 475                 480

Glu Arg Arg Ala Ala Leu Gly Gly Tyr Leu Pro Glu Arg Arg Glu Asn
                485                 490                 495

Tyr Asp Pro Ile Gln Val Pro Pro Leu Asp Lys Leu Arg Ser Val Arg
            500                 505                 510

Lys Gly Ser Gly Lys Gln Gln Ile Ala Thr Thr Met Ala Thr Val Arg
        515                 520                 525

Thr Phe Lys Glu Leu Met Arg Asp Lys Gly Leu Ala Asp Arg Leu Val
    530                 535                 540

Pro Ile Ile Pro Asp Glu Ala Arg Thr Phe Gly Leu Asp Ser Trp Phe
545                 550                 555                 560

Pro Thr Leu Lys Ile Tyr Asn Pro His Gly Gln Asn Tyr Val Pro Val
                565                 570                 575

Asp His Asp Leu Met Leu Ser Tyr Arg Glu Ala Pro Glu Gly Gln Ile
            580                 585                 590

Leu His Glu Gly Ile Asn Glu Ala Gly Ser Val Ala Ser Phe Ile Ala
        595                 600                 605

Ala Gly Thr Ser Tyr Ala Thr His Gly Lys Ala Met Ile Pro Leu Tyr
    610                 615                 620

Ile Phe Tyr Ser Met Phe Gly Phe Gln Arg Thr Gly Asp Ser Ile Trp
625                 630                 635                 640

Ala Ala Ala Asp Gln Met Ala Arg Gly Phe Leu Leu Gly Ala Thr Ala
                645                 650                 655

Gly Arg Thr Thr Leu Thr Gly Glu Gly Leu Gln His Met Asp Gly His
            660                 665                 670

Ser Pro Val Leu Ala Ser Thr Asn Glu Gly Val Glu Thr Tyr Asp Pro
        675                 680                 685

Ser Phe Ala Tyr Glu Ile Ala His Leu Val His Arg Gly Ile Asp Arg
    690                 695                 700

Met Tyr Gly Pro Gly Lys Gly Glu Asp Val Ile Tyr Tyr Ile Thr Ile
705                 710                 715                 720

Tyr Asn Glu Pro Thr Pro Gln Pro Ala Glu Pro Glu Gly Leu Asp Val
                725                 730                 735

Glu Gly Leu His Lys Gly Ile Tyr Leu Tyr Ser Arg Gly Glu Gly Thr
            740                 745                 750

Gly His Glu Ala Asn Ile Leu Ala Ser Gly Val Gly Met Gln Trp Ala
        755                 760                 765

Leu Lys Ala Ala Ser Ile Leu Glu Ala Asp Tyr Gly Val Arg Ala Asn
    770                 775                 780

Ile Tyr Ser Ala Thr Ser Trp Val Asn Leu Ala Arg Asp Gly Ala Ala
785                 790                 795                 800

Arg Asn Lys Ala Gln Leu Arg Asn Pro Gly Ala Asp Ala Gly Glu Ala
                805                 810                 815

Phe Val Thr Thr Gln Leu Lys Gln Thr Ser Gly Pro Tyr Val Ala Val
            820                 825                 830

Ser Asp Phe Ser Thr Asp Leu Pro Asn Gln Ile Arg Glu Trp Val Pro
        835                 840                 845

Gly Asp Tyr Thr Val Leu Gly Ala Asp Gly Phe Gly Phe Ser Asp Thr
    850                 855                 860

Arg Pro Ala Ala Arg Arg Phe Phe Asn Ile Asp Ala Glu Ser Ile Val
865                 870                 875                 880

Val Ala Val Leu Asn Ser Leu Ala Arg Glu Gly Lys Ile Asp Val Ser
                885                 890                 895

Val Ala Ala Gln Ala Ala Glu Lys Phe Lys Leu Asp Asp Pro Thr Ser
            900                 905                 910

Val Ser Val Asp Pro Asn Ala Pro Glu Glu
        915                 920

<210>  5
<211>  1263
<212>  DNA
<213>  Corynebacterium glutamicum

<220>
<221>  CDS
<222>  (1)..(1263)
<223>  lysC-Wildtypgen

<400>  5
gtg gcc ctg gtc gta cag aaa tat ggc ggt tcc tcg ctt gag agt gcg       48
Met Ala Leu Val Val Gln Lys Tyr Gly Gly Ser Ser Leu Glu Ser Ala
1               5                   10                  15

gaa cgc att aga aac gtc gct gaa cgg atc gtt gcc acc aag aag gct       96
Glu Arg Ile Arg Asn Val Ala Glu Arg Ile Val Ala Thr Lys Lys Ala
            20                  25                  30

gga aat gat gtc gtg gtt gtc tgc tcc gca atg gga gac acc acg gat      144
Gly Asn Asp Val Val Val Val Cys Ser Ala Met Gly Asp Thr Thr Asp
        35                  40                  45

gaa ctt cta gaa ctt gca gcg gca gtg aat ccc gtt ccg cca gct cgt      192
Glu Leu Leu Glu Leu Ala Ala Ala Val Asn Pro Val Pro Pro Ala Arg
    50                  55                  60

gaa atg gat atg ctc ctg act gct ggt gag cgt att tct aac gct ctc     240
Glu Met Asp Met Leu Leu Thr Ala Gly Glu Arg Ile Ser Asn Ala Leu
65                  70                  75                  80

gtc gcc atg gct att gag tcc ctt ggc gca gaa gcc caa tct ttc acg     288
Val Ala Met Ala Ile Glu Ser Leu Gly Ala Glu Ala Gln Ser Phe Thr
                85                  90                  95

ggc tct cag gct ggt gtg ctc acc acc gag cgc cac gga aac gca cgc     336
Gly Ser Gln Ala Gly Val Leu Thr Thr Glu Arg His Gly Asn Ala Arg
            100                 105                 110

att gtt gat gtc act cca ggt cgt gtg cgt gaa gca ctc gat gag ggc     384
Ile Val Asp Val Thr Pro Gly Arg Val Arg Glu Ala Leu Asp Glu Gly
        115                 120                 125

aag atc tgc att gtt gct ggt ttc cag ggt gtt aat aaa gaa acc cgc     432
Lys Ile Cys Ile Val Ala Gly Phe Gln Gly Val Asn Lys Glu Thr Arg
    130                 135                 140

gat gtc acc acg ttg ggt cgt ggt ggt tct gac acc act gca gtt gcg     480
Asp Val Thr Thr Leu Gly Arg Gly Gly Ser Asp Thr Thr Ala Val Ala
145                 150                 155                 160

ttg gca gct gct ttg aac gct gat gtg tgt gag att tac tcg gac gtt     528
Leu Ala Ala Ala Leu Asn Ala Asp Val Cys Glu Ile Tyr Ser Asp Val
                165                 170                 175

gac ggt gtg tat acc gct gac ccg cgc atc gtt cct aat gca cag aag     576
Asp Gly Val Tyr Thr Ala Asp Pro Arg Ile Val Pro Asn Ala Gln Lys
            180                 185                 190

ctg gaa aag ctc agc ttc gaa gaa atg ctg gaa ctt gct gct gtt ggc     624
Leu Glu Lys Leu Ser Phe Glu Glu Met Leu Glu Leu Ala Ala Val Gly
        195                 200                 205

tcc aag att ttg gtg ctg cgc agt gtt gaa tac gct cgt gca ttc aat     672
Ser Lys Ile Leu Val Leu Arg Ser Val Glu Tyr Ala Arg Ala Phe Asn
    210                 215                 220

gtg cca ctt cgc gta cgc tcg tct tat agt aat gat ccc ggc act ttg     720
Val Pro Leu Arg Val Arg Ser Ser Tyr Ser Asn Asp Pro Gly Thr Leu
225                 230                 235                 240

att gcc ggc tct atg gag gat att cct gtg gaa gaa gca gtc ctt acc     768
Ile Ala Gly Ser Met Glu Asp Ile Pro Val Glu Glu Ala Val Leu Thr
                245                 250                 255

ggt gtc gca acc gac aag tcc gaa gcc aaa gta acc gtt ctg ggt att     816
Gly Val Ala Thr Asp Lys Ser Glu Ala Lys Val Thr Val Leu Gly Ile
            260                 265                 270

tcc gat aag cca ggc gag gct gcg aag gtt ttc cgt gcg ttg gct gat     864
Ser Asp Lys Pro Gly Glu Ala Ala Lys Val Phe Arg Ala Leu Ala Asp
        275                 280                 285

gca gaa atc aac att gac atg gtt ctg cag aac gtc tct tct gta gaa     912
Ala Glu Ile Asn Ile Asp Met Val Leu Gln Asn Val Ser Ser Val Glu
    290                 295                 300

gac ggc acc acc gac atc acc ttc acc tgc cct cgt tcc gac ggc cgc      960
Asp Gly Thr Thr Asp Ile Thr Phe Thr Cys Pro Arg Ser Asp Gly Arg
305                 310                 315                 320

cgc gcg atg gag atc ttg aag aag ctt cag gtt cag ggc aac tgg acc      1008
Arg Ala Met Glu Ile Leu Lys Lys Leu Gln Val Gln Gly Asn Trp Thr
                325                 330                 335

aat gtg ctt tac gac gac cag gtc ggc aaa gtc tcc ctc gtg ggt gct      1056
Asn Val Leu Tyr Asp Asp Gln Val Gly Lys Val Ser Leu Val Gly Ala
            340                 345                 350

ggc atg aag tct cac cca ggt gtt acc gca gag ttc atg gaa gct ctg      1104
Gly Met Lys Ser His Pro Gly Val Thr Ala Glu Phe Met Glu Ala Leu
        355                 360                 365

cgc gat gtc aac gtg aac atc gaa ttg att tcc acc tct gag att cgt      1152
Arg Asp Val Asn Val Asn Ile Glu Leu Ile Ser Thr Ser Glu Ile Arg
    370                 375                 380

att tcc gtg ctg atc cgt gaa gat gat ctg gat gct gct gca cgt gca      1200
Ile Ser Val Leu Ile Arg Glu Asp Asp Leu Asp Ala Ala Ala Arg Ala
385                 390                 395                 400

ttg cat gag cag ttc cag ctg ggc ggc gaa gac gaa gcc gtc gtt tat      1248
Leu His Glu Gln Phe Gln Leu Gly Gly Glu Asp Glu Ala Val Val Tyr
                405                 410                 415

gca ggc acc gga cgc                                                  1263
Ala Gly Thr Gly Arg
            420

<210>  6
<211>  421
<212>  PRT
<213>  Corynebacterium glutamicum

<400>  6

Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45

Glu Leu Leu Glu Leu Ala Ala Ala Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Glu Ser Leu Gly Ala Glu Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Gly Arg Val Arg Glu Ala Leu Asp Glu Gly
        115                 120                 125

Lys Ile Cys Ile Val Ala Gly Phe Gln Gly Val Asn Lys Glu Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys
            180                 185                 190

Leu Glu Lys Leu Ser Phe Glu Glu Met Leu Glu Leu Ala Ala Val Gly
        195                 200                 205

Ser Lys Ile Leu Val Leu Arg Ser Val Glu Tyr Ala Arg Ala Phe Asn
    210                 215                 220

Val Pro Leu Arg Val Arg Ser Ser Tyr Ser Asn Asp Pro Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Val Leu Gly Ile
            260                 265                 270

Ser Asp Lys Pro Gly Glu Ala Ala Lys Val Phe Arg Ala Leu Ala Asp
        275                 280                 285

Ala Glu Ile Asn Ile Asp Met Val Leu Gln Asn Val Ser Ser Val Glu
    290                 295                 300

Asp Gly Thr Thr Asp Ile Thr Phe Thr Cys Pro Arg Ser Asp Gly Arg
305                 310                 315                 320

Arg Ala Met Glu Ile Leu Lys Lys Leu Gln Val Gln Gly Asn Trp Thr
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Glu Phe Met Glu Ala Leu
        355                 360                 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420

## Beschreibung

Gegenstand der Erfindung ist ein Verfahren zur Herstellung organisch-chemischer Verbindungen unter Verwendung von coryneformen Bakterien, in denen das für die Pyruvat-Dehydrogenase kodierende aceE-Gen abgeschwächt vorliegt, und diese Bakterien

### Stand der Technik

Chemische Verbindungen, mit denen insbesondere L-Aminosäuren, Ketosäuren, Vitamine, Nukleoside und Nukleotide und D-Aminosäuren gemeint sind, finden in der Humanmedizin, in der pharmazeutischen Industrie, in der Kosmetik, in der Lebensmittelindustrie und in der Tierernährung Anwendung.

Zahlreiche dieser Verbindungen werden durch Fermentation von Stämmen coryneformer Bakterien, insbesondere Corynebacterium glutamicum, hergestellt. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

In Wildtypstämmen verhindern strikte Regulationsmechanismen, die über den Eigenbedarf hinausgehende Produktion von Stoffwechselprodukten wie Aminosäuren und deren Abgabe ins Medium. Die Konstruktion von aus Herstellersicht als Aminosäureproduzenten bezeichneter Stämme erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder das Valin-Analogon 2-Thiazolalanin und chemische Verbindungen beispielsweise L-Aminosäuren wie L-Lysin oder L-Valin produzieren.

Seit einigen Jahren werden ebenfalls Methoden der rekombinanten DNA-Technik zur gezielten Stammverbesserung L-Aminosäure produzierender Stämme von Corynebacterium glutamicum eingesetzt, indem man einzelne Aminosäure-Biosynthesegene verstärkt oder abschwächt und die Auswirkung auf die Produktion der chemischen Verbindung untersucht.

Zusammenfassende Darstellungen zur Biologie, Genetik und Biotechnologie von Corynebacterium glutamicum sind im "Handbook of Corynebacterium glutamicum" (Eds.: L. Eggeling und M. Bott, CRC Press, Taylor & Francis, 2005), in der Spezialausgabe des Journal of Biotechnolgy (Chief Editor: A. Pühler) mit dem Titel "A New Era in Corynebacterium glutamicum Biotechnology" (Journal of Biotechnolgy 104/1-3, (2003)) und im Buch von T. Scheper (Managing Editor) "Microbial Production of L-Amino Acids" (Advances in Biochemical Engineering/Biotechnology 79, Springer Verlag, Berlin, Deutschland, 2003) zu finden.

Die Nukleotidsequenz des Genoms von Corynebacterium glutamicum ist bei Ikeda und Nakagawa (Applied Microbiology and Biotechnology 62, 99-109 (2003)), in der EP 1 108 790 und bei Kalinowski et al. (Journal of Biotechnolgy 104/1-3, (2003)) beschrieben.

Die Nukleotidsequenzen des Genoms von Corynebacterium glutamicum sind ebenfalls in der Datenbank des National Center for Biotechnology Information (NCBI) der National Library of Medicine (Bethesda, MD, USA), in der DNA Data Bank of Japan (DDBJ, Mishima, Japan) oder in der Nukleotidsequenz-Datenbank der European Molecular Biologies Laboratories (EMBL, Heidelberg, Deutschland bzw. Cambridge, UK) verfügbar.

Der besseren Übersichtlichkeit halber ist die Nukleotidsequenz des für die Pyruvat-Dehydrogenase-Untereinheit des Pyruvat-Dehydrogenase Komplex aus Corynebacterium glutamicum kodierenden aceE-Gens gemäß den Angaben der NCBI-Datenbank in SEQ ID NO:1 und die sich daraus ergebende Aminosäuresequenz der kodierten Pyruvat-Dehydrogenase in SEQ ID NO:2 und 4 dargestellt. In SEQ ID NO:3 sind stromaufwärts (upstream) und stromabwärts (downstream) gelegene Nukleotidsequenzen zusätzlich angegeben.

### Aufgabe der Erfindung

Die Erfinder haben sich die Aufgabe gestellt, neue Grundlagen für verbesserte Verfahren zur fermentativen Herstellung von organisch-chemischen Verbindungen, mit coryneformen Bakterien bereitzustellen.

### Beschreibung der Erfindung

Gegenstand der Erfindung ist ein Verfahren zur Herstellung einer organisch-chemischen Verbindung, dadurch gekennzeichnet, dass es einen oder mehrere Schritte enthält: Fermentation eines coryneformen Bakteriums, in dem das für die E1p Untereinheit des Pyruvat-Dehydrogenase Komplexes kodierenden aceE-Gen abgeschwächt vorliegt, wobei das Bakterium in einem geeignetem Nährmedium die gewünschte organisch-chemische Verbindung vermehrt herstellt im Vergleich zu einem coryneformen Bakterium, in dem das aceE-Gen nicht abgeschwächt vorliegt, und in der Zelle und/oder im Fermentationsmedium akkumuliert.

Daran schließt sich im Allgemeinen das Sammeln (collecting) der in der Fermentationsbrühe oder in den Zellen der Bakterien akkumulierten organisch-chemischen Verbindung an.

Die in der Fermentationsbrühe enthaltenen Verbindungen können aufkonzentriert und wenn gewünscht isoliert werden, wobei gegebenenfalls Bestandteile der Fermentationsbrühe und/oder die während der Fermentation gebildete Biomasse (Zellmasse) in ihrer Gesamtheit oder Teilen davon (> bis 100%) im Produkt verbleiben.

Mit dem Begriff "organisch-chemische Verbindung" sind organische Säuren, Vitamine, Nukleoside und Nukleotide und Aminosäuren und deren Salze gemeint. Weiterhin gehören dazu von Pyruvat abgeleitete Metabolite. Bei den organischen Säuren werden Ketosäuren, insbesondere Brenztraubensäure (Pyruvat), Bernsteinsäure (Succinat), Ketoisovaleriansäure und Ketoisocapronsäure, bevorzugt. Bei den Aminosäuren werden L-Aminosäuren, insbesondere die proteinogenen L-Aminosäuren und L-Homoserin, bevorzugt.

Unter "proteinogenen Aminosäuren" versteht man die Aminosäuren die in natürlichen Proteinen, das heißt in Proteinen von Mikroorganismen, Pflanzen, Tieren und Menschen vorkommen. Sie dienen als Struktureinheiten für Proteine, in denen sie über Peptidbindungen miteinander verknüpft sind.

Die Begriffe Protein und Polypeptid sind gegenseitig austauschbar.

Werden im folgenden proteinogene L-Aminosäuren erwähnt, sind damit eine oder mehrere der Aminosäuren einschließlich ihrer Salze, ausgewählt aus der Gruppe L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin und L-Prolin gemeint. Besonders bevorzugt sind die L-Aminosäuren L-Aspartat, L-Lysin, L-Homoserin, L-Threonin, L-Methionin, L-Alanin, L-Valin und L-Leucin. Ganz besonders bevorzugt sind L-Lysin und L-Valin.

Wird im Folgenden L-Lysin oder Lysin erwähnt, sind damit nicht nur die Basen, sondern sind auch die Salze wie z.B. Lysin-Monohydrochlorid oder Lysin-Sulfat gemeint.

Der Begriff "Abschwächung" bzw. "Abschwächen" beschreibt in diesem Zusammenhang die Verringerung oder Ausschaltung der intrazellulären Aktivität eines oder mehrerer Enzyme bzw. Proteine in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym bzw. Protein inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt. Unter einem "Ausgangsmikroorganismus" versteht man den Mikroorganismus, in dem die Abschwächung des aceE-Gens durchgeführt wird.

Die erfindungsgemäß erzeugten rekombinanten Mikroorganismen sind ebenso Teil der Erfindung.

Der Pyruvat-Dehydrogenase Komplex katalysiert die Umwandlung von Brenztraubensäure zu Kohlendioxid, NADH und Acetyl-Coenzym A. Der Pyruvat-Dehydrogenase Komplex besteht aus drei Enzymen bzw. Untereinheiten, die jeweils eine Teilreaktion katalysieren. Die als E1 oder E1p bezeichnete Untereinheit katalysiert die Thiaminpyrophospat-abhängige oxidative Decarboxylierung von Pyruvat. Die E1p-Untereinheit wird auch als Pyruvat-Dehydrogenase bezeichnet (EC No. 1.2.4.1). Eine allgemeine Darstellung zum Pyruvat-Dehydrogenase Komplex findet sich beispielsweise im Lehrbuch von G. Gottschalk "Bacterial Metabolism" (Second Edition, Springer Verlag, New York, USA, 1986). Angaben zum Pyruvat-Dehydrogenase-Komplex von Corynebacterium glutamicum finden sich auf Seite 245 bis 249 des oben erwähnten "Handbook of Corynebacterium glutamicum" und bei Schreiner et al. (Journal of Bacteriology 187(17), 6005-6018 (2005) .

Das erfindungsgemäße Verfahren zur Herstellung von organisch-chemischen Verbindungen wird unter Verwendung von coryneformen Bakterien durchgeführt. Bei den coryneformen Bakterien wird die Gattung Corynebacterium bevorzugt. Diese beruhen besonders bevorzugt auf organisch-chemische Verbindungen ausscheidenden Elternstämmen, zu denen folgenden Arten gehören:
Corynebacterium efficiens, wie zum Beispiel der Stamm DSM44549,
Corynebacterium glutamicum, wie zum Beispiel der Stamm ATCC13032,
Corynebacterium callunae, wie zum Beispiel der Stamm ATCC15991,
Corynebacterium thermoaminogenes wie zum Beispiel der Stamm FERM BP-1539, und
Corynebacterium ammoniagenes, wie zum Beispiel der Stamm ATCC6871,
wobei die Art Corynbacterium glutamicum ganz besonders bevorzugt wird.

Einige Vertreter der Art Corynebacterium glutamicum, die zu den/die Elternstämme gehören, sind im Stand der Technik auch unter anderen Artbezeichnungen bekannt. Hierzu gehören beispielsweise:
Corynebacterium acetoacidophilum ATCC13870,
Corynebacterium lilium DSM20137,
Corynebacterium melassecola ATCC17965,
Brevibacterium flavum ATCC14067,
Brevibacterium lactofermentum ATCC13869, und
Brevibacterium divaricatum ATCC14020.

Angaben zur taxonomischen Einordnung von Stämmen dieser Gruppe von Bakterien findet man unter anderem bei Seiler (Journal of General Microbiology 129, 1433-1477 (1983), Kämpfer und Kroppenstedt (Canadian Journal of Microbiology 42, 989-1005 (1996)), Liebl et al (International Journal of Systematic Bacteriology 41, 255-260 (1991) und in der US-A-5,250,434.

Die für die Massnahme der Abschwächung eingesetzten coryneformen Bakterien besitzen bevorzugt bereits die Fähigkeit die organisch-chemische Verbindung in der Zelle anzureichern oder in das sie umgebende Nährmedium auszuscheiden und zu akkumulieren. Insbesondere haben die eingesetzten coryneformen Bakterien die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l der organisch-chemischen Verbindung in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern.

Bekannte Vertreter von Stämme coryneformer Bakterien, die die Fähigkeit besitzen L-Lysin zu produzieren, sind beispielsweise die Stämme
Corynebacterium glutamicum DM58-1/pDM6 (= DSM4697)
beschrieben in EP 0 358 940,
Corynebacterium glutamicum MH20 (= DSM5714) beschrieben in EP 0 435 132,
Corynebacterium glutamicum AHP-3 (= FermBP-7382) beschrieben in EP 1 108 790, und
Corynebacterium thermoaminogenes AJ12521 (= FERM BP-3304) beschrieben in US 5,250,423.

Bekannte Vertreter von Stämmen coryneformer Bakterien, die die Fähigkeit besitzen L-Valin zu produzieren, sind beispielsweise die Stämme
Brevibacterium lactofermentum FERM BP-1763
beschrieben in US 5,188,948,
Brevibacterium lactofermentum FERM BP-3007
beschrieben in US 5,521,074,
Corynebacterium glutamicum FERM BP-3006
beschrieben in US 5,521,074, und
Corynebacterium glutamicum FERM BP-1764
beschrieben in US 5,188,948.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Die genannten Stämme von Corynebacterium thermoaminogenes (FERM BP-1539, FERM BP-1540, FERM BP-1541 und FERM BP-1542) sind in der US-A 5,250,434 beschrieben.

Bei dem für die Massnahmen der Erfindung verwendeten aceE-Gen handelt es sich um ein Polynukleotid ausgewählt aus der Gruppe Polynukleotid, das vor der Massnahme der Abschwächung für ein Polypeptid kodiert, dessen Aminosäuresequenz ≥ (mindestens) 90%, bevorzugt ≥ 95%, besonders bevorzugt ≥ 98% und ganz besonders bevorzugt ≥ 99% identisch ist mit der Aminosäuresequenz von SEQ ID NO: 2 und das die Aktivität einer Pyruvat-Dehydrogenase besitzt.

Polynukleotid, das vor der Massnahme der Abschwächung eine Nukleotidsequenz umfasst, die ≥ (mindestens) 90%, bevorzugt ≥ 95%, besonders bevorzugt ≥ 98% und ganz besonders bevorzugt ≥ 99% identisch ist mit der Nukleotidsequenz von SEQ ID NO: 1 und die für ein Polypeptid kodiert, das die Aktivität einer Pyruvat-Dehydrogenase besitzt.

Polynukleotid, das vor der Massnahme der Abschwächung eine Nukleotidsequenz umfasst die unter stringenten Hybridisierungsbedingungen mit der zu SEQ ID NO:1 komplementären Nukleotidsequenz hybridisiert und die für ein Polypeptid kodiert, das die Aktivität einer Pyruvat-Dehydrogenase besitzt.

Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO:1 komplementäre Nukleotidsequenz und die Zielsequenz, d. h. die mit der Sonde behandelten Polynukleotide, mindestens 90% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 90% Identität zur Nukleotidequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 90% oder mindestens 91%, bevorzugt mindestens 92% oder mindestens 93% oder mindestens 94% oder mindestens 95% oder mindestens 96% und ganz besonders bevorzugt mindestens 97% oder mindestens 98% oder mindestens 99% Identität zur Sequenz der eingesetzten Sonde besitzen und für ein Polypeptid mit Pyruvat-Dehydrogenase Enzymaktivität kodieren. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Die so erhaltenen Nukleotidsequenzen kodieren für Polypeptide mit Pyruvat-Dehydrogenase Enzymaktivität, die mindestens 90% bevorzugt mindestens 92% oder mindestens 94% oder mindestens 96%, und ganz besonders bevorzugt mindestens 97% oder mindestens 98% oder mindestens 99% identisch sind mit der Aminosäuresequenz von SEQ ID NO:2.

Polynukleotid, das vor der Massnahme der Abschwächung eine Nukleotidsequenz umfasst, die für ein Polypeptid mit Pyruvat-Dehydrogenase Aktivität kodiert und die mit der Nukleotidsequenz eines Polynukleotids identisch ist, das durch eine Polymerasekettenreaktion (PCR) unter Verwendung von DNA isoliert aus einem coryneformen Bakterium und unter Verwendung eines Primerpaars erhältlich ist, deren Nukleotidsequenzen jeweils mindestens 15, mindestens 18, mindestens 21 oder mindestens 24 aufeinanderfolgende Nukleotide umfassen, die aus der Nukleotidsequenz zwischen Position 1 und 500 von SEQ ID NO:3 und aus der komplementären Nukleotidsequenz zwischen Position 3267 und 3769 von SEQ ID NO:3 ausgewählt werden. Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994). Weitere Details zur PCR-Technik sind in der DE 199 43 587 beschrieben.

Der Primer kann mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Polynukleotid, das vor der Massnahme der Abschwächung für ein Polypeptid mit Pyruvat-Dehydrogenase Aktivität und mit der Aminosäuresequenz von SEQ ID NO: 2 kodiert. Gegebenenfalls enthält das kodierte Polypeptid einen (1) oder mehrere konservative Aminosäuraustausch(e). Bevorzugt enthält das Polypeptid höchstens zwei (2), höchstens drei (3), höchstens vier (4)oder höchstens fünf (5) konservative Aminosäureaustausche.

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Aparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Polynukleotid, das vor der Massnahme der Abschwächung die Nukleotidsequenz von SEQ ID NO: 1 umfasst.

Die genannten Polynukleotide sind typischerweise in den Chromosomen der genannten coryneformen Bakterien vorhanden.

Zur Erzielung einer Abschwächung können entweder die Expression der Gene oder die katalytischen Eigenschaften der Genprodukte herabgesetzt oder ausgeschaltet werden. Gegebenenfalls werden beide Maßnahmen kombiniert.

Die Genexpression kann durch geeignete Kulturführung oder durch genetische Veränderung (Mutation) der Signalstrukturen der Genexpression verringert werden. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z.B. in der Patentanmeldung WO 96/15246, bei Boyd und Murphy (Journal of Bacteriology 170: 5949-5952 (1988)), bei Voskuil und Chambliss (Nucleic Acids Research 26: 3584-3590 (1998), bei Pätek et al. (Microbiology 142: 1297-309 (1996) und Journal of Biotechnology 104: 311-323 (2003)) und in bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Ein Beispiel für die gezielte Regulation der Genexpression ist die Klonierung des abzuschwächenden Gens unter die Kontrolle eines durch Zugabe dosierter Mengen von IPTG (Isopropyl-β-D-thiogalactopyranosid) induzierbaren Promotors wie zum Beispiel des trc-Promotors oder des tac-Promotors. Hierzu eignen sich Vektoren wie beispielsweise der Escherichia coli Expressionsvektor pXK99E (WO0226787; hinterlegt gemäß Budapester Vertrag am 31. Juli 2001 in DH5alpha/pXK99E als DSM14440 bei der Deutschen Sammlung für Mikroorganismen und Zellkulturen (DSMZ, Braunschweig, Deutschland)) oder pVWEx2 (Wendisch, Ph. D. thesis, Berichte des Forschungszentrums Jülich, Jül-3397, ISSN 0994-2952, Jülich, Deutschland (1997)), die eine IPTGabhängige Expression des klonierten Gens in Corynebacterium glutamicum ermöglichen.

Eingesetzt wurde diese Methode beispielsweise in der Patentschrift WO0226787 zur regulierten Expression des deaD-Gens durch Integration des Vektors pXK99EdeaD in das Genom von Corynebacterium glutamicum und von Simic et al. (Applied and Environmental Microbiology 68: 3321-3327 (2002)) zur regulierten Expression des glyA-Gens durch Integration des Vektors pK18mobglyA' in Corynebacterium glutamicum.

Eine weitere Methode zur spezifischen Verringerung der Genexpression ist die Antisense-Technik, wobei kurze Oligodesoxnyukleotide oder Vektoren zur Synthese längerer Antisense-RNA in die Zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs,Jül-2906, ISSNO9442952, Jülich, Deutschland, 1994) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von ≥ 0 bis 75%, ≥ 0 bis 50%, ≥ 0 bis 25%, ≥ 0 bis 10% oder ≥ 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die genannten Massnahmen zur werden vorzugsweise im 5'-terminalen Teil der Kodierregion durchgeführt, welcher für den N-Terminus des Polypeptids kodiert. Bezeichnet man die Gesamtlänge eines Polypeptids (gemessen als Anzahl der chemisch verbundenen L-Aminosäuren) mit 100%, so gehört - im Rahmen der vorliegenden Erfindung - zum N-Terminus des Polypeptids der Teil der Aminosäuresequenz, welcher, gerechnet ab der Start-Aminosäure L-Formyl-Methionin 80% der nachfolgenden L-Aminosäuren enthält.

Eine Methode zur Bestimmung der enzymatischen Aktivität der Pyruvat-Dehydrogenase ist bei Schreiner et al. (Journal Bacteriology 187(17), 6005-6018 (2005)) beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Eine gebräuchliche Methode, Gene von C. glutamicum zu mutieren, ist die von Schwarzer und Pühler (Bio/Technology 9, 84-87 (1991)) beschriebene Methode der Gen-Unterbrechung ("gene disruption") und des Gen-Austauschs ("gene replacement").

Bei der Methode der Gen-Unterbrechung wird zum Beispiel ein zentraler Teil der Kodierregion des interessierenden Gens in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob, pK19mob, pK18mobsacB oder pK19mobsacB (Schäfer et al., Gene 145, 69-73 (1994)), pGEM-T (Promega Corporation, Madison, WI, USA), pCR2.1-TOPO (Shuman (1994), Journal of Biological Chemistry 269:32678-84; US-Patent 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173: 4510-4516) in Frage. Der Plasmidvektor, der den zentralen Bereich der Kodierregion des Gens enthält, wird anschließend durch Konjugation oder Transformation in den gewünschten Stamm von C. glutamicum überführt. Die Methode der Konjugation ist beispielsweise bei Schäfer et al. (Journal of Bacteriology 172: 1663-1666 (1990) und Applied and Environmental Microbiology 60: 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben.

Nach homologer Rekombination mittels eines "cross-over"-Ereignisses wird die Kodierregion des betreffenden Gens durch die Vektorsequenz unterbrochen und man erhält zwei unvollständige Allele, denen jeweils das 3'- bzw. das 5'-Ende fehlt. Diese Methode wurde beispielsweise von Fitzpatrick et al. (Applied Microbiology and Biotechnology 42, 575-580 (1994)) zur Ausschaltung des recA-Gens von C. glutamicum verwendet.

Bei der Methode des Genaustausches ("gene replacement") wird eine Mutation wie z.B. eine Deletion, Insertion oder Basenaustausch in dem interessierenden Gen in-vitro hergestellt. Das hergestellte Allel wird wiederum in einen für C. glutamicum nicht replikativen Vektor kloniert und dieser anschließend durch Transformation oder Konjugation in den gewünschten Wirt von C. glutamicum überführt. Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation bzw. des Allels. Diese Methode ist bei Schwarzer und Pühler (Bio/Technology 9: 84-87 (1991) beschrieben und wurde beispielsweise von Peters-Wendisch et al. (Microbiology 144, 915 - 927 (1998)) verwendet, um das pyc-Gen von C. glutamicum durch eine Deletion auszuschalten oder von Wehmeier et al. (Microbiology 144: 1853-1862 (1998)) zum Einfügen einer Deletion in das rel-Gen von C. glutamicum.

Einen Überblick über verschiedene gentechnische Methoden bei C. glutamicum geben Kirchner und Tauch (Journal of Biotechnology 104: 287-299 (2003).

Für den Einbau einer Deletion in das aceE-Gen von Corynebacterium glutamicum ist beispielsweise der von Schreiner et al. (Journal of Bacteriology 187(17), 6005-6018 (2005)) beschriebene Vektor pK19mutaceE geeignet.

Zusätzlich kann es für die verbesserte Produktion von L-Aminosäuren vorteilhaft sein, in den auf die beschriebene Weise hergestellten coryneformen Bakterien eines oder mehrere Enzyme des jeweiligen Biosyntheseweges, der Glykolyse, der Anaplerotik, des Pentosephosphat-Zyklus, des Aminosäure-Exports und gegebenenfalls regulatorische Proteine überzuexprimieren. Die Verwendung endogener Gene wird ebenso wie bei den beschriebenen Massnahmen der Abschwächung im allgemeinen bevorzugt.

Unter "endogenen Genen" oder "endogenen Nukleotidsequenzen" versteht man die in der Population einer Art vorhandenen Gene beziehungsweise Nukleotidsequenzen oder Allele.

So kann für die Herstellung von L-Lysin eines oder mehrere der Gene überexprimiert werden, ausgewählt aus der Gruppe:
Ein für ein Dihydrodipicolinat-Synthase kodierendes Gen dapA, wie beispielsweise das in der EP 0 197 335 beschriebene dapA-Gen des Wildtyps von Corynebacterium glutamicum.
Ein für eine Glucose-6-Phosphat Dehydrogenase kodierendes Gen zwf, wie beispielsweise das in der JP-A-09224661 und EP-A-1108790 beschriebene zwf-Gen des Wildtyps von Corynebacterium glutamicum.

Die in der US-2003-0175911-A1 beschriebenen zwf-Allele von Corynebacterium glutamicum, die für ein Protein kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist.

Die in der WO 2005/058945 beschriebenen zwf-Allele von Corynebacterium glutamicum, die für ein Protein kodieren, bei dem beispielsweise das L-Serin an Position 8 der Aminosäuresequenz durch L-Threonin ersetzt ist oder bei dem das L-Glycin an Position 321 durch L-Serin ersetzt ist.

Ein für eine Pyruvat-Carboxylase kodierendes Gen pyc, wie beispielsweise das in der DE-A-198 31 609 und EP 1108790 beschriebene pyc-Gen des Wildtyps von Corynbebacterium glutamicum.

Das für das in der EP 1 108 790 beschriebene pyc-Allel von Corynebacterium glutamicum, das für ein Protein kodiert, bei dem L-Prolin an Position 458 der Aminosäuresequenz durch L-Serin ersetzt ist.

Die in der WO 02/31158 und insbesondere EP1325135B1 beschriebene pyc-Allele von Corynebacterium glutamicum, die für Proteine kodieren, welche einen oder mehrere der Aminosäureaustausche ausgewählt aus der Gruppe L-Valin an Position 1 ersetzt durch L-Methionin, L-Glutaminsäure an Position 153 ersetzt durch L-Asparaginsäure, L-Alanin an Position 182 ersetzt durch L-Serin, L-Alanin an Position 206 ersetzt durch L-Serin, L-Histidin an Position 227 ersetzt durch L-Arginin, L-Alanin an Position 455 ersetzt durch Glycin und L-Asparaginsäure an Position 1120 ersetzt durch L-Glutaminsäure tragen.

Ein für eine Aspartatkinase kodierendes lysC Gen wie beispielsweise das als SEQ ID NO:281 in der EP-A-1108790 (Siehe auch Zugangsnummer AX120085 und 120365) und das als SEQ ID NO:25 in der WO 01/00843 (Siehe Zugangsnummer AX063743) beschriebene von lysC-Gen des Wildtyps von Corynebacterium glutamicum

Ein für eine feed back resistente Aspartatkinase Variante kodierendes lysC^{FBR} Allel.

Unter "feed back" resistenten Aspartatkinasen versteht man Aspartatkinasen, die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene beziehungsweise Allele werden auch als lysC^{FBR}-Allele bezeichnet. Im Stand der Technik (Tabelle 1) sind zahlreiche lysC^{FBR}-Allele beschrieben, die für Aspartatkinase-Varianten kodieren, welche Aminosäureaustausche im Vergleich zum Wildtypprotein besitzen. Die Kodierregion des Wildtyp lysC-Gens von Corynebacterium glutamicum entsprechend der Zugangsnummer AX756575 der NCBI Datenbank ist in SEQ ID NO:5 und das von diesem Gen kodierte Protein in SEQ ID NO:6 dargestellt

### Tabelle 1

| lysC^{FBR}-Allele kodierend für feed back resistente Aspartatkinasen | | | |
|---|---|---|---|
| Bezeichnung des Allels | Weitere Angaben | Referenz | Zugangsnummer |
| lysC^{FBR}-E05108 | | JP 1993184366-A (Sequenz 1) | E05108 |
| lysC^{FBR}-E06825 | lysC A279T | JP 1994062866-A (Sequenz 1) | E06825 |
| lysC^{FBR}-E06826 | lysC A279T | JP 1994062866-A (Sequenz 2) | E06826 |
| lysC^{FBR}-E06827 | | JP 1994062866-A (Sequenz 3) | E06827 |
| lysC^{FBR}-E08177 | | JP 1994261766-A (Sequenz 1) | E08177 |
| lysC^{FBR}-E08178 | lysC A279T | JP 1994261766-A (Sequenz 2) | E08178 |
| lysC^{FBR}-E08179 | lysC A279V | JP 1994261766-A (Sequenz 3) | E08179 |
| lysC^{FBR}-E08180 | lysC S301F | JP 1994261766-A (Sequenz 4) | E08180 |
| lysC^{FBR}-E08181 | lysC T308I | JP 1994261766-A (Sequenz 5) | E08181 |
| lysC^{FBR}-E08182 | | JP 1994261766-A (Sequenz 6) | E08182 |
| lysC^{FBR}-E12770 | | JP 1997070291-A (Sequenz 13) | E12770 |
| lysC^{FBR}-E14514 | | JP 1997322774-A (Sequenz 9) | E14514 |
| lysC^{FBR}-E16352 | | JP 1998165180-A (Sequenz 3) | E16352 |
| lysC^{FBR}-E16745 | | JP 1998215883-A (Sequenz 3) | E16745 |
| lysC^{FBR}-E16746 | | JP 1998215883-A (Sequenz 4) | E16746 |
| lysC^{FBR}-I74588 | lysC S317A | US 5688671-A (Sequenz 1) | I74588 |
| lysC^{FBR}-I74589 | lysC A279T | US 5688671-A (Sequenz 2) | I74589 |
| lysC^{FBR}-I74590 | | US 5688671-A (Sequenz 7) | 174590 |
| lysC^{FBR}-I74591 | lysC A279T | US 5688671-A (Sequenz 8) | I74591 |
| lysC^{FBR}-I74592 | | US 5688671-A (Sequenz 9) | I74592 |
| lysC^{FBR}-I74593 | lysC A279T | US 5688671-A (Sequenz 10) | I74593 |
| lysC^{FBR}-I74594 | | US 5688671-A (Sequenz 11) | I74594 |
| lysC^{FBR}-I74595 | lysC A279T | US 5688671-A (Sequenz 12) | I74595 |
| lysC^{FBR}-I74596 | | US 5688671-A (Sequenz 13) | I74596 |
| lysC^{FBR}-I74597 | lysC A279T | US 5688671-A (Sequenz 14) | 174597 |
| lysC^{FBR}-X57226 | lysC S301Y | EP0387527 Kalinowski et al., Molecular and General Genetics 224:317-324 (1990) | X57226 |
| lysC^{FBR}-L16848 | lysC G345D | Follettie and Sinskey NCBI Nucleotide Database (1990) | L16848 |
| lysC^{FBR}-L27125 | lysC R320G lysC G345D | Jetten et al., Applied Microbiology Biotechnology 43:76-82 (1995) | L27125 |
| lysC^{FBR} | lysC T311I | WO0063388 (Sequenz 17) | |
| lysC^{FBR} | lysC S301F | US3732144 | |
| lysC^{FBR} | lysC S381F | EP0435132 | |
| lysC^{FBR} | lysC S317A | US5688671 (Sequenz 1) | |
| lysC^{FBR} | lysC T380I | WO 01/49854 | |

Bevorzugt werden folgende lysC^{FBR}-Allele: lysC A279T (Austausch von Alanin an Position 279 des kodierten Aspartatkinaseproteins gemäß SEQ ID NO: 6 gegen Threonin), lysC A279V (Austausch von Alanin an Position 279 des kodierten Aspartatkinaseproteins gemäß SEQ ID NO: 6 gegen Valin), lysC S301F (Austausch von Serin an Position 301 des kodierten Aspartatkinaseproteins gemäß SEQ ID NO: 6 gegen Phenylalanin), lysC T308I (Austausch von Threonin an Position 308 des kodierten Aspartatkinaseproteins gemäß SEQ ID NO: 6 gegen Isoleucin), lysC S301Y (Austausch von Serin an Position 308 des kodierten Aspartatkinaseproteins gemäß SEQ ID NO: 6 gegen Tyrosin), lysC G345D (Austausch von Glycin an Position 345 des kodierten Aspartatkinaseproteins gemäß SEQ ID NO: 6 gegen Asparaginsäure), lysC R320G (Austausch von Arginin an Position 320 des kodierten

Aspartatkinaseproteins gemäß SEQ ID NO: 6 gegen Glycin), lysC T311I (Austausch von Threonin an Position 311 des kodierten Aspartatkinaseproteins gemäß SEQ ID NO: 6 gegen Isoleucin), lysC S381F (Austausch von Serin an Position 381 des kodierten Aspartatkinaseproteins gemäß SEQ ID NO: 16 gegen Phenylalanin), lysC S317A (Austausch von Serin an Position 317 des kodierten Aspartatkinaseproteins gemäß SEQ ID NO: 6 gegen Alanin) und lysC T380I (Austausch von Threonin an Position 380 des kodierten Aspartatkinaseproteins gemäß SEQ ID NO: 6 gegen Isoleucin).

Besonders bevorzugt werden das lysC^{FBR}-Allel lysC T311I (Austausch von Threonin an Position 311 des kodierten Aspartatkinaseproteins gemäß SEQ ID NO: 6 gegen Isoleucin) und ein lysC^{FBR}-Allel enthaltend mindestens einen Austausch ausgewählt aus der Gruppe A279T (Austausch von Alanin an Position 279 des kodierten Aspartatkinaseproteins gemäß SEQ ID NO: 6 gegen Threonin) und S317A (Austausch von Serin an Position 317 des kodierten Aspartatkinaseproteins gemäß SEQ ID NO: 6 gegen Alanin).

Ein für ein Lysin-Export-Protein kodierendes Gen lysE, wie beispielsweise das in der DE-A-195 48 222 beschriebene lysE-Gen von des Wildtyps Corynebacterium glutamicum.

Ein für eine Diaminopimelate-Dehydrogenase kodierendes Gen ddh, wie beispielsweise das in der EP 1 108 790 beschriebene ddh-Gen des Wildtyps Corynebacterium glutamicum.

Das für das Zwa1-Protein kodierende Gen zwa1 des Wildtyps von Corynebacterium glutamicum (US 6,632,644).

So kann für die Herstellung von L-Valin eines oder mehrere der Gene überexprimiert werden, ausgewählt aus der Gruppe:

Für Acetolactate Synthase (EC 4.1.3.18) kodierenden Gene ilvBN, wie beispielsweise die von Keilhauer et al. (Journal of Bacteriology 175(17):5595-603 (1993) oder die in der EP1108790 beschriebenen ilvBN Gene von Corynebacterium glutamicum (Siehe auch Zugangsnummern L09232 und AX127147).

Ein für eine feed back resistente Acetolactate Synthase (EC 4.1.3.18) kodierendes ilvBN^{FBR} Allele, wie beispielsweise das in der EP1491634 beschriebene ilvBN^{FBR} Allel.

Ein für eine Isomeroreductase (EC 1.1.1.86) kodierendes Gen ilvC, wie beispielsweise das von Keilhauer et al. (Journal of Bacteriology 175(17):5595-603 (1993) oder das in der EP1108790 beschriebene ilvC-Gen (Siehe auch Zugangsnummern C48648 und AX127147).

Ein für eine Dihydroxy-acid Dehydratase (EC 4.2.1.9) kodierendes ilvD-Gen, wie beispielsweise das in der EP1006189 beschriebene ilvD-Gen (Siehe auch Zugangsnummer AX136925).

Ein für eine Transaminase B (EC 2.6.1.42) kodierendes ilvE-Gen, wie beispielsweise das in der EP1108790 beschriebene ilvE-Gen (Siehe auch Zugangsnummern AX127150 und AX122498).

Für Proteine, die den L-Valin-Export aus der Zelle katalysieren, kodierende Gene brnEF, wie beispielsweise die in der EP1096010 beschriebenen brnEF Gene.

Ein für eine Phosphoenolpyruvat Carboxykinase kodierendes pck-Gen, wie beispielsweise das in der EP1094111 und EP1108790beschriebene pck-Gen.

Ein für ein Malat-Enzym (malic enzyme, EC:1.1.1.40) kodierendes mez-Gen, wie beispielsweise das in der EP1108790 beschriebene mez-Gen.

Unter Überexpression versteht man allgemein eine Erhöhung der intrazellulären Konzentration oder Aktivität einer Ribonukleinsäure, eines Proteins oder eines Enzyms.

Die genannte Erhöhung der Konzentration oder Aktivität eines Genproduktes lässt sich beispielsweise dadurch erzielen, dass man die Kopienzahl der entsprechenden Polynukleotide um mindestens eine Kopie erhöht.

Eine weit verbreitete Methode zur Erhöhung der Kopienzahl besteht darin, dass man das entsprechende Gen oder Allel in einen Vektor, bevorzugt ein Plasmid, einbaut, der von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al. (Journal of Biotechnology 104, 27-40 (2003)).

Eine andere verbreitete Methode zur Erzielung einer Überexpression ist das Verfahren der chromosomalen Genamplifikation. Bei dieser Methode wird mindestens eine zusätzliche Kopie des interessierenden Gens oder Allels in das Chromosom eines coryneformen Bakteriums eingefügt.

In einer Ausführungsform, wie sie beispielsweise bei Reinscheid et al. (Applied and Environmental Microbiology 60, 126-132) für das hom-thrB-Operon beschrieben ist, wird ein in C. glutamicum nicht-replikatives Plasmid, welches das interessierende Gen enthält, in ein coryneformes Bakterium überführt. Nach homologer Rekombination mittels eines "cross over"-Ereignisses enthält der resultierende Stamm mindestens zwei Kopien des betreffenden Gens beziehungsweise Allels.

In einer anderen Ausführungsform, die in der WO 03/040373 und US-2003-0219881-A1 beschrieben ist, wird eine oder mehrere Kopie(n) des interessierenden Gens mittels mindestens zweier Rekombinationsereignisse in einen gewünschten Ort des Chromosoms von C. glutamicum eingefügt. Auf diese Weise wurde beispielsweise eine Kopie eines lysC-Allels, das für eine L-Lysin insensitive Aspartatkinase kodiert, in das gluB-Gen von C. glutamicum eingebaut.

In einer weiteren Ausführungsform, die in der WO 03/014330 und US-2004-0043458-A1 beschrieben ist, wird mittels mindestens zweier Rekombinationsereignisse am natürlichen Ort mindestens eine weitere Kopie, vorzugsweise in tandem-Anordnung zu dem bereits vorhandenen Gen oder Allel, des interessierenden Gens eingebaut. Auf diese Weise wurde beispielsweise eine tandem-Duplikation eines lysC^{FBR}-Allels am natürlichen lysC-Genort erzielt.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in dem Übersichtsartikel von Patek et al. (Journal of Biotechnology 104(1-3), 311-323 (2003) beschrieben. Weiterhin können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des betreffenden Gens, typischerweise im Abstand von ungefähr 1 - 500 Nukleotiden vom Startkodon, eines coryneformen Bakteriums eingefügt werden. Es ist ebenfalls möglich das Gen mit einem Promotor zu verknüpfen und die erhaltene Expressionseinheit in ein extrachromosomal replizierendes Plasmid oder in das Chromosom eines coryneformen Bakteriums einzubauen.

Darüberhinaus kann die Promotor- und Regulationsregion oder die Ribosomenbindungsstelle, die sich stromaufwärts des Strukturgens befindet, mutiert werden. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Alternativ kann weiterhin eine Überexpression des betreffenden Gens oder Allels durch Veränderung der Medienzusammensetzung und Kulturführung erreicht werden.

Durch die Maßnahmen der Überexpression wird die Aktivität oder Konzentration des entsprechenden Proteins/Polypeptids im allgemeinen um mindestens 10%, 25%, 50%, 75%, 100%, 150%, 200%, 300%, 400% oder 500%, maximal bis 1000% oder 2000%, bezogen auf die Aktivität oder Konzentration des Proteins im Ausgangs-Mikroorganismus beziehungsweise Elternstammes erhöht. Unter einem Ausgangs-Mikroorganismus oder Elternstamm versteht man einen Mikroorganismus, an dem die Massnahme der Überexpression oder Verstärkung des aceE-Gens durchgeführt werden.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 111: 2630-2647 (1999)) bestimmt werden.

Schließlich kann es für die Produktion von organisch-chemischen Verbindungen, vorteilhaft sein, neben der Abschwächung des aceE-Gens unerwünschte Nebenreaktionen auszuschalten ( Nakayama: "Breeding of Amino Acid Producing Microorganisms", in: Overproduction of Microbial Products, Krumphanzl, Sikyta, Vanek (eds.), Academic Press, London, UK, 1982).

So kann für die Herstellung von L-Lysin eines oder mehrere der Gene abgeschwächt werden, ausgewählt aus der Gruppe:

Ein für eine Homoserin-Dehydrogenase kodierendes hom-Gen, wie beispielsweise in der EP0555661 oder in der EP1108790 beschrieben.

Ein für eine Malat-Chinon Oxidoreduktase kodierendes mqo-Gen, wie beispielsweise in der WO 02/086137 oder wie in der US-Anmeldung 60/645588 (entsprechend DE-Anmeldung 102005032429) beschrieben.

Ein für eine Citratsynthase kodierendes g1tA-Gen, wie beispielsweise das in der EP09992892 oder EP1108790 beschriebene gltA-Gen.

Ein für eine Phosphoenolpyruvat-Carboxykinase kodierendes pckA-Gen, wie beispielsweise in der EP1094111 beschrieben.

Ein für ein Malat-Enzym (malic enzyme, EC:1.1.1.40) mez-Gen, wie beispielsweise in der EP1367130 beschrieben.

So kann für die Herstellung von L-Valin eines oder mehrere der Gene abgeschwächt werden, ausgewählt aus der Gruppe:

Ein für eine Pyruvat-Carboxylase kodierendes pyc-Gen. Das pyc-Gen von Corynebacterium glutamicum ist in der EP1015621 beschrieben.

Ein für eine Pyruvat-Oxidase kodierendes poxB-Gen, wie beispielsweise in der EP1096013 beschrieben.

Ein für eine Phosphoenolpyruvat-Carboxylase kodierendes ppc-Gen. Das ppc-Gen von Corynebacterium glutamicum ist beispielsweise bei O'Regan et al. (Gene 77, 237-251 (1989)) beschrieben.

Ein für eine Threonin Deaminase kodierendes ilvA-Gen. Das ilvA-Gen von Corynebacterium glutamicum ist in der beispielsweise in der EP1108790 beschrieben.

Ein für ein Valin-Import-Protein kodierendes brnQ-Gen wie beispielsweise in der WO 03/023044 beschrieben.

Eine weitere Verbesserung der Ketoisovaleriansäure Herstellung kann erzielt werden durch Abschwächung eines oder mehrerer der Gene ausgewählt aus der Gruppe:

Ein für eine Transaminase B (EP 1 217 069) kodierendes ilvE-Gen.

Ein für eine Threonin Deaminase kodierendes ilvA-Gen.

Ein für eine 2-Isopropylmalat Synthase kodierendes leuA-Gen (Patek et al. 1994, Applied and Environmental Microbiology 60: 133-140).

Ein für eine 3-Isopropylmalat Dehydrogenase kodierendes leuB-Gen (Patek et al. 1998, Applied Microbiology and Biotechnology 50: 42-47).

Ein für die grosse Untereinheit der Isopropylmalat Isomerase kodierendes leuC-Gen (Gross et al., Biochemistry 2 1042-1052 (1963)).

Ein für die kleine Untereinheit der Isopropylmalat Isomerase kodierendes leuD-Gen (Gross et al., Biochemistry 2 1042-1052 (1963)).

Ein für eine alpha-Ketoisovaleriansäure Hydroxymethyltransferase kodierendes panB-Gen (Sahm und Eggeling, Applied and Environmental Microbiology 65(5):1973-1979 (1999)).

Ein für eine Pantothenat Synthetase kodierendes panC-Gen (Sahm und Eggeling, Applied and Environmental Microbiology 65(5):1973-1979 (1999)) .

Weiterhin kann eine Verbesserung der Ketoisovaleriansäure-Herstellung erzielt werden durch Überexpression eines oder mehrerer der Gene ausgewählt aus der Gruppe:

Ein für eine IlvB-Untereinheit der Acetohydroxysäure Synthase kodieriendes ilvB-Gen (Keilhauer et al. 1993, Journal of Bacteriology 175: 5595-5603).

Ein für eine IlvN-Untereinheit der Acetohydroxysäure Synthase kodieriendes ilvN-Gen (Keilhauer et al. 1993, Journal of Bacteriology 175: 5595-5603).

Ein für eine Isomeroreduktase kodierendes ilvC-Gen (Keilhauer et al. 1993, Journal of Bacteriology 175: 5595-5603).

Ein für eine Dihydroxysäure Dehydratase kodierendes ilvD-Gen (EP 1 006 189).

Ein für eine Phosphoenolpyruvat-Carboxykinase kodierendes pckA-Gen.

Ein für ein Malat-Enzym (malic enzyme, EC:1.1.1.40) mez-Gen.

Eine gemeinsame Überexpression der Gene ilvB, ilvN, ilvC und ilvD kann beispielsweise mit Hilfe des in EP 1 006 189 beschriebenen Plasmids pECM3ilvBNCD erzielt werden. Dieses Plasmid ist in Form des Escherichia coli K12 Stamm DH5αmcr/pECM3ilvBNCD als DSM12457 bei der DSMZ hinterlegt.

Eine weitere Verbesserung der Keto-Isocapronsäure Produktion kann erzielt werden durch Überexpression eines oder mehrerer der Gene ausgewählt aus der Gruppe:

Ein für eine IlvB-Untereinheit der Acetohydroxysäure Synthase kodieriendes ilvB-Gen (Keilhauer et al. 1993, Journal of Bacteriology 175: 5595-5603).

Ein für eine IlvN-Untereinheit der Acetohydroxysäure Synthase kodieriendes ilvN-Gen (Keilhauer et al. 1993, Journal of Bacteriology 175: 5595-5603).

Ein für eine Isomeroreduktase kodierendes ilvC-Gen (Keilhauer et al. 1993, Journal of Bacteriology 175: 5595-5603).

Ein für eine Dihydroxysäure Dehydratase kodierendes ilvD-Gen (EP 1 006 189).

Ein für eine 2-Isopropylmalat Synthase kodierendes leuA-Gen (Patek et al. 1994, Applied and Environmental Microbiology 60: 133-140).

Ein für eine 3-Isopropylmalat Dehydrogenase kodierendes leuB-Gen (Patek et al. 1998, Applied Microbiology and Biotechnology 50: 42-47).

Ein für die grosse Untereinheit der Isopropylmalat Isomerase kodierendes leuC-Gen (Gross et al., Biochemistry 2 1042-1052 (1963)).

Ein für die kleine Untereinheit der Isopropylmalat Isomerase kodierendes leuD-Gen (Gross et al., Biochemistry 2 1042-1052 (1963)).

Eine weitere Verbesserung der Keto-Isocapronsäure Produktion kann erzielt werden durch Abschwächung eines oder mehrerer der Gene ausgewählt aus der Gruppe:

Ein für eine alpha-Ketoisovaleriansäure Hydroxymethyltransferase kodierendes panB-Gen (Sahm und Eggeling, Applied and Environmental Microbiology 65(5):1973-1979 (1999)) .

Ein für eine Pantothenat Synthetase kodierendes panC-Gen (Sahm und Eggeling, Applied and Environmental Microbiology 65(5):1973-1979 (1999)).

Die für die organisch-chemische Verbindungen produzierenden coryneformen Bakterien beschriebenen Zusatzmassnahmen der Überexpression und Abschwächung können beliebig miteinander kombiniert werden. So kann beispielsweise im Falle von L-Lysin produzierenden coryneformen Bakterien gleichzeitig das lysE-Gen überexprimiert und das gltA-Gen abgeschwächt werden.

Die Leistung der hergestellten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert. Mit dem Begriff "Produkt" ist die gewünschte organisch-chemische Verbindung gemeint.

Die hergestellten coryneformen Bakterien können kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von organisch-chemischen Verbindungen, beispielsweise L-Aminosäuren, kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben-oder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden. Diese Stoffe können einzeln oder als Mischung verwendet werden. Bevorzugt werden Mischungen bestehend aus Zucker und Essigsäure.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Dem Kulturmedium können überdies geeignete Vorstufen der jeweiligen organisch-chemischen Verbindung, beispielsweise die Vorstufe einer Aminosäure, zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten organisch-chemischen Verbindung, beispielsweise Aminosäure, gebildet hat. Dieses Ziel wird normalerweise innerhalb von 10 Stunden bis 160 Stunden erreicht. Bei kontinuierlichen

Verfahren sind längere Kultivierungszeiten möglich. Durch die Tätigkeit der Bakterien kommt es zu einer Anreicherung der gewünschten organisch-chemischen Verbindung im Fermentationsmedium und/oder in den Zellen der Bakterien.

Beispiele für geeignete Fermentationsmedien finden sich unter anderem in den Patentschriften 5,770,409, US 5,840,551 und US 5,990,350 oder US 5,275,940.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Die auf diese Weise hergestellte Fermentationsbrühe wird anschließend zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medium enthält/enthalten sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte organisch-chemische Verbindung beispielsweise eine L-Aminosäure wie L-Lysin oder L-Valin c) die im Laufe der Fermentation gebildeten organischen Nebenprodukte und d) die durch die Fermentation nicht verbrauchten Bestandteile des eingesetzten Fermentationsmediums beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten organisch-chemischen Verbindung erzeugt und gegebenenfalls ausgeschieden werden. Hierzu gehören auch Zucker wie zum Beispiel Trehalose.

Im Falle der Aminosäure L-Lysin sind im Stand der Technik im wesentlichen vier verschiedene Produktformen bekannt.

Eine Gruppe L-Lysin haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen die L-Aminosäure aus der Fermentationsbrühe gesammelt, isoliert oder gereinigt wird, um das L-Aminosäure haltige Produkt oder die gereinigte L-Aminosäure herzustellen.

Zur Herstellung von festen, reinen L-Aminosäuren werden im wesentlichen Methoden der Ionenaustauschchromatographie gegebenfalls unter Verwendung von Aktivkohle und Methoden der Kristallisierung angewendet.Im Falle des L-Valin erhält man auf diese Weise die weitgehend reine Verbindung. Im Falle des Lysin erhält man auf diese Weise die entsprechende Base oder ein entsprechendes Salz wie beispielsweise das Monohydrochlorid (Lys-HCl) oder das Lysinsulfat (Lys₂-H₂SO₄).

Im Falle des Lysin ist in der EP-B-0534865 ein Verfahren zur Herstellung wässriger, basischer L-Lysin haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerbares und weitgehend staubfreies Produkt überführt werden.

Der Begriff "staubfrei" bedeutet, daß das Produkt lediglich geringe Anteile (< 5 %) an Körngrößen unter 100 µm Durchmesser enthält.

"Lagerbar", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann ohne das ein wesentlicher Verlust (< 5%) der jeweiligen Aminosäure auftritt.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu behandeln so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub d. h. Partikeln mit einer Korngrösse < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Im Falle des Lysin wird bei der Herstellung Lysin-haltiger Produkte das Verhältnis der Ionen bevorzugt so eingestellt, dass das Ionenverhältnis entsprechend nachstehender Formel

2x[SO₄²⁻]+[Cl⁻]-[NH₄⁺]-[Na⁺]-[K⁺]-2x[Mg²⁺]-2x[Ca²⁺]/[L-Lys]

0,68 bis 0,95, bevorzugt 0,68 bis 0,90 ergibt so wie von Kushiki et al. in der US 20030152633 beschrieben.

Im Falle des Lysin hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-% , 72 Gew.-%, 73 Gew.-% sind ebenfalls möglich.

Im Falle einer elektrisch neutralen Aminosäure wie dem L-Tryptophan hat das auf diese Weise hergestellte feste Produkt auf Fermentationsbrühebasis einen Aminosäuregehalt von mindestens 5 Gew.-%, 10 Gew.-%, 20 Gew.-%, 30 Gew.-% und maximal 50 Gew.-%, 60 Gew.-%, 70 Gew.-%, 80 Gew.-%, 90 Gew.-% oder bis 95 Gew.-%.

Der Wassergehalt des festen Produktes beträgt bis zu 5 Gew.-%, bevorzugt bis zu 4 Gew.-%, und besonders bevorzugt weniger als 3 Gew.-%.

### Anwendungsbeispiel 1: Valinproduktion

### Stammkonstruktion

Das Plasmid pECKAilvBNC (Elisakova et al., 2005. Applied and Environmental Microbiology 71:207-213) wurde nach Birnboim (Birnboim, H. C. 1983. Methods in Enzymology 100:243-255) aus E. coli DH5α präpariert. Die Herstellung elektrokompetenter Zellen der Stämme C. glutamicum ATCC 13032 und C. glutamicum ATCC 13032_ΔaceE (Schreiner et al., 2005. Journal of Bacteriology 187:6005-6018), sowie die Transformation beider Stämme mit dem Plasmid pECKAilvBNC erfolgte nach Van der Rest et al. (Applied Microbiology and Biotechnology 52:541-545, 1999).

Zur Kontrolle wurde das Plasmid pECKAilvBNC aus den Stämmen C. glutamicum ATCC 13032/pECKAilvBNC und C. glutamicum ATCC 13032_ΔaceE/pECKAilvBNC nach Eikmanns et al. (Microbiology 140:1817-1828, 1994) isoliert und mit dem aus E. coli DH5α präparierten Plasmid in einem 0,8 %igen Agarosegel verglichen.

Fermentation der Stämme C. glutamicum ATCC 13032/pECKAilvBNC und
C. glutamicum ATCC 13032_ΔaceE/pECKAilvBNC

Zur Anzucht von C. glutamicum ATCC 13032/pECKAilvBNC und C. glutamicum ATCC 13032_ΔaceE/pECKAilvBNC wurde Zellsuspension mit einer sterilen Impföse aus einer Glycerinkultur entnommen und auf einer trypton yeast - Agarplatte (Trypton 16 g/l; Hefeextrakt 10 g/l; NaCl 5 g/l; Agar 15 g/l) mit Kanamycin (50 µg/ml) ausgestrichen. Die Platten für den Stamm C. glutamicum ATCC 13032_ΔaceE/pECKAilvBNC enthielten zusätzlich 0,5 % (w/v) Kaliumacetat (KAc). Nach Inkubation der Agarplatten bei 30 °C für 48 Stunden wurde eine Kolonie für das Animpfen von 5 ml trypton yeast - Medium (Trypton 16 g/l; Hefeextrakt 10 g/l; NaCl 5 g/l) supplementiert mit 0,5 % (w/v) KAc und Kanamycin (50 µg/ml) verwendet. Nach 6 h Inkubation wurden die kompletten 5 ml in 50 ml Medium gleicher Zusammensetzung überführt und über Nacht inkubiert. Für die Valin-Fermentation wurden die Zellen durch Zentrifugation (Mit Centrifuge 5804 R, Eppendorf-Netheler-Hinz GmbH, Köln, bei 5000 rpm; 4 °C; 10 min) sedimentiert, mit 20 ml 0,9 % (w/v) NaCl gewaschen und in CgXII-Medium (Keilhauer et al., 1993. Journal of Bacteriology 175:5595-5603) supplementiert mit 4 % (w/v) Glukose, 1 % (w/v) KAc, 0,5 % (w/v) Hirn-Herz-Bouillon und Kanamycin (50 µg/ml) inokuliert. Das Kultivieren von C. glutamicum erfolgte unter aeroben Bedingungen (500 ml Erlenmeyerkolben mit 2 Schikanen) auf einem Rotationsschüttler für 48 h bei 120 rpm und 30 °C.

### Analytik

Das Wachstum der Kulturen wurde photometrisch (Ultrospec 3000, Amersham Pharmacia Biotech GmbH, Freiburg) anhand der optischen Dichte bei einer Wellenlänge von 600 nm bestimmt. Es wurde 1 ml Zellsuspension zentrifugiert (In Tischzentrifuge vom Typ 5415D, (Eppendorf, Hamburg) bei 13000 rpm, 10 min, Raumtemperatur) und der Überstand für die Bestimmung der Substrat - und Produktkonzentrationen verwendet. Die Bestimmung der Valin- und Alaninkonzentrationen erfolgte mittels reversed-phase high pressure liquid chromatography (HP 1100, Hewlett-Packard, Waldbronn, Deutschland) mit einem Fluoreszenzdetektor nach automatischer Vorsäulenderivatisierung mit ortho-Phthaldialdehyd (OPA) (Lindroth und Mopper, 1979. Analytical Chemistry. 51:1667-1674). Die Glukose-Konzentrationen wurde indirekt durch Messung der NADPH-Konzentration nach enzymatischer Umsetzung der Glukose mit Glukose-Kinase und Glukose-6-Phosphat-Dehydrogenase bestimmt (Roche Diagnostics, Penzberg, Deutschland).

### Ergebnisse

Die Ergebnisse der Fermentation von C. glutamicum ATCC 13032/pECKAilvBNC und C. glutamicum ATCC 13032_ΔaceE/pECKAilvBNC sind in Tabelle 1 und 2 dargestellt.

**Tabelle 1:**

| C. glutamicum ATCC 13032/pECKAilvBNC | | | |
|---|---|---|---|
| Zeit [h] | OD₆₀₀ | Valin [mM] | Alanin [mM] |
| 0 | 1,1 | 0 | 0 |
| 48 | 70 | 4,6 | 0 |

**Tabelle 2:**

| C. glutamicum ATCC 13032_ΔaceE/pECKAilvBNC | | | |
|---|---|---|---|
| Zeit [h] | OD₆₀₀ | Valin [mM] | Alanin [mM] |
| 0 | 1,1 | 0 | 0 |
| 48 | 29,6 | 53,3 | 5,2 |

### Anwendungsbeispiel 2: Lysinproduktion

### Stammkonstruktion

Im Stamm C. glutamicum DM1729 (Georgi et al., 2005. Metabolic Engineering 7:291-301) wurde das aceE-Gen nach Schreiner et al. (Journal of Bacteriology 187:6005-6018, 2005) deletiert. Der Stamm DM1729 wurde als DSM17576 am 16.September 2005 bei der Deutschen Sammlung für Mikroorganismen und Zellkulturen (DSMZ, Braunschweig, Deutschland) nach Budapester Vertrag hinterlegt. Die Inaktivierung des Pyruvat-Dehydogenase-Komplexes (PDHC) in C. glutamicum DM1729_ΔaceE wurde durch Messung der spezifischen PDHC-Aktivität (Schreiner et al. 2005) bestätigt.

### Fermentation der Stämme C. glutamicum DM1729 und C. glutamicum DM1729 ΔaceE

Zur Anzucht von C. glutamicum DM1729 und C. glutamicum DM1729_ΔaceE wurde Zellsuspension mit einer sterilen Impföse aus einer Glycerinkultur entnommen und auf einer Hirn-Herz - Agarplatte (37 g/l Hirn-Herz-Bouillon, 15 g/l Agar) ausgestrichen. Die Agarplatten für den Stamm C. glutamicum DM1729_ΔaceE enthielten zusätzlich 0,5 % (w/v) Kaliumacetat (KAc). Nach Inkubation der Agarplatten bei 30 °C für 2 Tage wurde eine Kolonie für das Animpfen von 5 ml Hirn-Herz - Medium supplementiert mit 0,5 % (w/v) KAc verwendet. Nach 6 h Inkubation wurden die kompletten 5 ml in 50 ml Medium gleicher Zusammensetzung überführt und über Nacht inkubiert. Für die Lysin-Fermentation wurden die Zellen durch Zentrifugation (Mit Centrifuge 5804 R, Eppendorf-Netheler-Hinz GmbH, Köln, bei 5000 rpm; 4 °C; 10 min) sedimentiert, mit 20 ml 0,9 % (w/v) NaCl gewaschen und in CgXII-Medium (Keilhauer et al., 1993. Journal of Bacteriology 175:5595-5603) supplementiert mit 4 % (w/v) Glukose, 1 % (w/v) KAc und 0,5 % (w/v) Hirn-Herz Bouillon inokuliert. Das Kultivieren von C. glutamicum erfolgte unter aeroben Bedingungen (500 ml Erlenmeyerkolben mit 2 Schikanen) auf einem Rotationsschüttler für 48 h bei 120 rpm bei 30 °C.

### Analytik

Das Wachstum der Kulturen wurde photometrisch (Ultrospec 3000, Amersham Pharmacia Biotech GmbH, Freiburg) anhand der optischen Dichte bei einer Wellenlänge von 600 nm bestimmt. Es wurde 1 ml Zellsuspension zentrifugiert (In Tischzentrifuge vom Typ 5415D (Eppendorf) bei 13000 rpm, 10 min, Raumtemperatur) und der Überstand für die Bestimmung der Substrat - und Produktkonzentrationen verwendet. Die Bestimmung der Lysinkonzentrationen erfolgte mittels "reversed-phase high pressure liquid chromatography" (RP-HPLC mit HP 1100, Hewlett-Packard, Waldbronn, Deutschland) mit einem Fluoreszenzdetektor nach automatischer Vorsäulenderivatisierung mit ortho-Phthaldialdehyd (OPA) (Lindroth und Mopper, 1979. Analytical Chemistry. 51:1667-1674). Die Glukose-Konzentrationen wurde indirekt durch Messung der NADPH-Konzentration nach enzymatischer Umsetzung der Glukose mit Glukose-Kinase und Glukose-6-Phosphat-Dehydrogenase bestimmt (Roche Diagnostics, Penzberg, Deutschland).

### Ergebnisse

Die Ergebnisse der Fermentation von C. glutamicum DM1729 und C. glutamicum DM1729_ΔaceE sind in Tabelle 3 und 4 dargestellt

**Tabelle 3:**

| C. glutamicum DM1729 | | |
|---|---|---|
| Zeit [h] | OD₆₀₀ | Lysin [mM] |
| 0 | 0,95 | 1,9 |
| 48 | 44,50 | 33,3 |

**Tabelle 4:**

| C. glutamicum DM1729_ΔaceE | | |
|---|---|---|
| Zeit [h] | OD₆₀₀ | Lysin [mM] |
| 0 | 0,85 | 1,9 |
| 48 | 17,60 | 49,3 |

### Anwendungsbeispiel 3: Succinatproduktion

### Stämme

Für die Succinat-Fermentationen wurden die Stämme C. glutamicum ATCC 13032 und C. glutamicum ATCC 13032 ΔaceE verwendet.

Fermentation der Stämme C. glutamicum ATCC 13032 und C. glutamicum ATCC 13032 ΔaceE

Zur Anzucht von C. glutamicum ATCC 13032 und C. glutamicum ATCC 13032 ΔaceE wurde Zellsuspension mit einer sterilen Impföse aus einer Glycerinkultur entnommen und auf einer 2xTY-Agarplatte ausgestrichen. Die Agarplatten für den Stamm C. glutamicum ΔaceE enthielten zusätzlich 0,5 % (w/v) Kaliumacetat (KAc). Nach Inkubation der Platten bei 30 °C für 2 Tage wurde eine Kolonie für das Animpfen von 5 ml 2xTY-Medium + 0,5 % (w/v) KAc verwendet. Nach 6 h Inkubation wurden die kompletten 5 ml in 50 ml Medium gleicher Zusammensetzung überführt und über Nacht inkubiert. Für die Succinat-Fermentation wurden die Zellen durch Zentrifugation (5000 rpm; 4 °C; 10 min) sedimentiert, mit 20 ml 0,9 % (w/v) NaCl gewaschen und in CgXII-Medium (Eikmanns et al, Microbiology 140:1817-1828 (1994)) + 4 % (w/v) Glucose + 1 % (w/v) KAc überführt. Das Kultivieren von C. glutamicum erfolgte in einem 4-fach Fermentationssystem der Firma DASGIP AG (Jülich, Deutschland. Dabei wurde bis zur 2h der Fermentation die Kultivierung aerob durchgeführt (0,5 vvm, 500 rpm) und nach 2h Fermentationsdauer wurde unter anaeroben Bedingungen (keine Begasung, 100 rpm) weiter fermentiert. Die Temperatur betrug während der gesamten Fermentation 30 °C.

### Analytik

Das Wachstum über die Zeit wurde anhand der optischen Dichte bei einer Wellenlänge von 600 nm verfolgt. Es wurde 1 ml Zellsuspension zentrifugiert (13000 rpm, 10 min, Raumtemperatur) und der Überstand für die Bestimmung der Succinatkonzentrationen verwendet. Die Bestimmung der Succinatkonzentrationen wurde mittels HPLC auf Aminophase mit Phosphat-Acetonitril-Puffer bestimmt.

### Ergebnisse

Die Ergebnisse der Fermentation von C. glutamicum ATCC 13032 und C. glutamicum ATCC13032 ΔaceE sind in Tabelle 5 und 6 dargestellt.

**Tabelle 5:**

| C. glutamicum ATCC 13032 | | |
|---|---|---|
| Zeit [h] | OD₆₀₀ | Succinat [mM] |
| 0 | 16, 9 | 0 |
| 48 | 18,7 | 38 |

**Tabelle 6:**

| C. glutamicum ATCC 13032 ΔaceE | | |
|---|---|---|
| Zeit [h] | OD₆₀₀ | Succinat [mM] |
| 0 | 15,7 | 0 |
| 48 | 15,5 | 52 |

### Anwendungsbeispiel 4: Ketoisovaleratproduktion

### Stammkonstruktion

In dem Stamm C. glutamicum ΔilvE (Marienhagen et al, Journal of Bacteriology. 187: 7639-7646 (2005)) wurde der Pyruvat-Dehydrogenase-Komplex durch Deletion des aceE-Gens nach Schreiner et al. (Journal of Bacteriology 187:6005-6018 (2005)) inaktiviert. Das Plasmid pJC1ilvBNCD (Sahm und Eggeling, Applied and Environmental. Microbiology 65: 1973-1979 (1999)) wurde nach Birnboim (Methods Enzymology 100:243-255 (1983)) aus E. coli DH5α/pJC1ilvBNCD präpariert. Die Herstellung elektrokompetenter Zellen des Stammes C. glutamicum ΔaceE ΔilvE sowie die Transformation dieses Stammes mit dem Plasmid pJC1ilvBNCD erfolgte nach Van der Rest et al. (Applied Microbiology and Biotechnology 52:541-545 (1999)). Zur Kontrolle wurde das Plasmid pJC1ilvBNCD aus dem Stamm C. glutamicum ΔaceE ΔilvE pJC1ilvBNCD nach Eikmanns et al. (Microbiology 140:1817-1828 (1994)) isoliert und mit dem aus E. coli DH5α/ pJC1ilvBNCD präparierten Plasmid in einem 0,8 %igen Agarosegel verglichen.

Fermentation der Stämme C. glutamicum ΔaceE, C. glutamicum ΔaceE ΔilvE und C. glutamicum ΔaceE ΔilvE pJC1ilvBNCD

Zur Anzucht von C. glutamicum ΔaceE und C. glutamicum ΔaceE ΔilvE wurde Zellsuspension mit einer sterilen Impföse aus einer Glycerinkultur entnommen und auf einer 2xTY-Agarplatte mit 0,5 % (w/v) Kaliumacetat (KAc) ausgestrichen. Zur Kultivierung von C. glutamicum ΔaceE ΔilvE pJC1ilvBNCD wurde dem Medium grundsätzlich Kanamycin (50 µg/ml) zugegeben. Nach Inkubation der Platten bei 30 °C für 2 Tage wurde eine Kolonie für das Animpfen von 5 ml 2xTY-Medium + 0,5 % (w/v) KAc verwendet. Nach 6 h

Inkubation wurden die kompletten 5 ml in 50 ml Medium gleicher Zusammensetzung überführt und über Nacht inkubiert. Für die Ketoisovalerat-Fermentationen wurden die Zellen durch Zentrifugation (5000 rpm; 4 °C; 10 min) sedimentiert, mit 20 ml 0,9 % (w/v) NaCl gewaschen und in CgXII-Medium + 4 % (w/v) Glucose + 1 % (w/v) KAc + 1 mM Isoleucin, 1mm Leucin, 1 mM Valin inokuliert. Das Kultivieren von C. glutamicum erfolgte unter aeroben Bedingungen (Erlenmeyerkolben mit Schikanen) auf einem Rotationsschüttler (120 rpm) bei 30 °C.

### Analytik

Das Wachstum über die Zeit wurde anhand der optischen Dichte bei einer Wellenlänge von 600 nm verfolgt. Es wurde 1 ml Zellsuspension zentrifugiert (13000 rpm, 10 min, Raumtemperatur) und der Überstand für die Bestimmung der Ketoisovaleratkonzentration verwendet. Diese erfolgte mittels reversed-phase high pressure liquid chromatographie (HP 1100, Hewlett-Packard, Waldbronn, Germany) mit einem Fluoreszenzdetektor nach Derivatisierung mit 1,2-Diamino-4,5-Dimethoxybenzol (DDB) (Hara et al Journal of Chromatography. 344: 33-39 (1985)).

### Ergebnisse

Die Ergebnisse der Fermentation von C. glutamicum ΔaceE, C. glutamicum ΔaceE ΔilvE und C. glutamicum ΔaceE ΔilvE pJC1ilvBNCD sind in der Tabelle 1, 2 und 3 zusammengefasst. In Fermentationen mit dem Wildtypstamm (ATCC 13032) konnte kein Ketoisovalerat nachgewiesen werden.

**Tabelle 7:**

| C. glutamicum ΔaceE | | |
|---|---|---|
| Zeit[h] | OD [600 nm] | 2-Ketoisovalerat [mM] |
| 0 | 1,1 | 0 |
| 48 | 32,2 | 15 |

**Tabelle 8:**

| C. glutamicum ΔaceE ΔilvE | | |
|---|---|---|
| Zeit[h] | OD [600 nm] | 2-Ketoisovalerat [mM] |
| 0 | 1,1 | 0 |
| 48 | 25,6 | 40 |

**Tabelle 9:**

| C. glutamicum ΔaceE ΔilvE pJC1ilvBNCD | | |
|---|---|---|
| Zeit[h] | OD [600 nm] | 2-Ketoisovalerat [mM] |
| 0 | 1,1 | 0 |
| 48 | 25,1 | 76 |

## Patentansprüche

1. Verfahren zur Herstellung einer organisch-chemischen Verbindung, **dadurch gekennzeichnet dass** es den folgenden Schritt enthält
Fermentation eines coryneformen Bakteriums, in dem das für die E1p Untereinheit des Pyruvat-Dehydrogenase Komplexes kodierende aceE-Gen abgeschwächt vorliegt, wobei das Bakterium in einem geeignetem Medium die genannte organisch-chemische Verbindung vermehrt herstellt und in der Zelle und/oder im Fermentationsmedium akkumuliert.

2. Verfahren nach Anspruch 1, **dadurch gekennzeichnet, dass** das aceE-Gen ausgeschaltet wird.

3. Verfahren nach Anspruch 1 oder 2, **dadurch gekennzeichnet, dass** das aceE-Gen vor der Abschwächung für ein Polypeptid kodiert, dessen Aminosäuresequenz zu mindestens 90% identisch ist mit SEQ ID NO: 2.

4. Verfahren nach nach Anspruch 1 oder 2, **dadurch gekennzeichnet, dass** das aceE-Gen vor der Abschwächung ein Polynukleotid umfasst, dessen Nukleotidsequenz zu mindestens 90% identisch ist mit SEQ ID NO: 1.

5. Verfahren nach einem oder mehreren der Ansprüche1 bis 4, **dadurch gekennzeichnet, dass** das aceE-Gen vor der Abschwächung ein Polynukleotid umfasst, das mit der zu SEQ ID NO:1 komplementären Nukleotidsequenz unter stringenten Bedingungen hybridisiert, wobei die stringenten Bdingungen einen Waschschritt mit einer Salzkonzentration entsprechend 2xSSC bis 0,5 x SSC bei einer Temperatur von 50 bis 68°C umfassen.

6. Verfahren nach Anspruch 3, **dadurch gekennzeichnet, dass** das aceE-Gen vor der Abschwächung für ein Polypeptid kodiert, dessen Aminosäuresequenz identisch ist mit SEQ ID NO: 2.

7. Verfahren nach Anspruch 4, **dadurch gekennzeichnet, dass** das aceE-Gen vor der Abschwächung ein Polynukleotid umfasst, dessen Nukleotidsequenz identisch ist mit SEQ ID NO: 1.

8. Verfahren nach Anspruch 1 oder 2, **dadurch gekennzeichnet, dass** das aceE-Gen vor der Abschwächung ein Polynukleotid umfasst, dessen Nukleotidsequenz mit der Nukleotidsequenz eines Polynukleotids identisch ist, das durch eine Polymerasekettenreaktion (PCR) unter Verwendung von DNA isoliert aus einem coryneformen Bakterium und unter Verwendung eines Primerpaars erhältlich ist, deren Nukleotidsequenzen jeweils mindestens 15, aufeinanderfolgende Nukleotide umfassen, die aus der Nukleotidsequenz zwischen Position 1 und 500 von SEQ ID NO:3 und aus der komplementären Nukleotidsequenz zwischen Position 3267 und 3769 von SEQ ID NO:3 ausgewählt werden.

9. Verfahren nach einem oder mehreren der Ansprüche 1 bis 8, **dadurch gekennzeichnet, dass** die Abschwächung des aceE-Gens durch eine oder mehrere der Massnahmen ausgewählt aus der Gruppe Deletion von mindesten einem Nukleotid, Insertion von mindestestens einem Nukleotid, Transition von mindestens einem Nukleotid mit Einbau einer Nichtsinnmutation und Transversion von mindestens einem Nukleotid mit Einbau einer Nichtsinnmutation hergestellt wird.

10. Verfahren nach einem oder mehreren der Ansprüche 1 bis 9, **dadurch gekennzeichnet, dass** das coryneforme Bakterium ein Bakterium, ausgewählt aus der Gruppe Corynebacterium efficiens, Corynebacterium glutamicum, Corynebacterium callunae, Corynebacterium thermoaminogenes und Corynebacterium ammoniagenes, ist.

11. Verfahren nach Anspruch 10, **dadurch gekennzeichnet, dass** das coryneforme Bakterium Corynebacterium glutamicum ist.

12. Verfahren nach einem oder mehreren der Ansprüche 1 bis 11, **dadurch gekennzeichnet, dass** es sich bei der organisch-chemischen Verbindung um eine L-Aminosäure handelt.

13. Verfahren nach Anspruch 12, **dadurch gekennzeichnet, dass** es sich um eine proteinogene L-Aminosäure oder um L-Homoserin handelt.

14. Verfahren nach Anspruch 13, **dadurch gekennzeichnet, dass** es sich bei der proteinogenen L-Aminosäure um eine oder mehrere der Aminosäuren, ausgewählt aus der Gruppe L-Aspartat, L-Lysin, L-Homoserin, L-Threonin, L-Methionin, L-Alanin, L-Valin und L-Leucin, handelt.

15. Verfahren nach Anspruch 14, **dadurch gekennzeichnet, dass** es sich bei der proteinogenen L-Aminosäure um L-Lysin handelt.

16. Verfahren nach Anspruch 14, **dadurch gekennzeichnet, dass** es sich bei der proteinogenen L-Aminosäure um L-Valin handelt.

17. Verfahren nach Anspruch 15, **dadurch gekennzeichnet, dass** in dem coryneformen Bakterium eines oder mehrere der Gene ausgewählt aus der Gruppe
a) ein für eine Dihydrodipicolinat-Synthase kodierendes dapA-Gen,
b) ein für eine Glucose-6-Phosphat Dehydrogenase kodierendes zwf-Gen oder Allel,
c) ein für eine Pyruvat-Carboxylase kodierendes pyc-Gen oder Allel,
d) ein für eine Aspartatkinase kodierendes lysC-Gen oder lysC^{FBR}-Allel,
e) ein für ein Lysin-Export-Protein kodierendes lysE-Gen,
f) ein für eine Diaminopimelate-Dehydrogenase kodierendes ddh-Gen, und
g) ein für ein zwa1-Protein kodierendes zwa1-Gen, verstärkt vorliegt (vorliegen).

18. Verfahren nach Anspruch 15 oder 17, **dadurch gekennzeichnet, dass** in dem coryneformen Bakterium eines oder mehrere der Gene ausgewählt aus der Gruppe
a) ein für eine Homoserin-Dehydrogenase kodierendes hom-Gen,
b) ein für eine Malat-Chinon Oxidoreduktase kodierendes mqo-Gen,
c) ein für eine Citratsynthase kodierends gltA-Gen,
d) ein für eine Phosphoenolpyruvat-Carboxykinase kodierendes pck-Gen, und
e) ein für ein Malat-Enzym kodierendes mez-Gen, abgeschwächt vorliegt (vorliegen).

19. Verfahren nach Anspruch 16, **dadurch gekennzeichnet, dass** in dem coryneformen Bakterium eines oder mehrere der Gene ausgewählt aus der Gruppe
a) für Acetolactate Synthase kodierende ilvBN-Gene oder ilvBN^{FBR}-Allele,
b) ein für eine Isomeroreduktase kodierendes ilvC-Gen,
c) ein für eine Dihydroxysäure Dehydratase kodierendes ilvD-Gen,
d) ein für eine Transaminase B kodierendes ilvE-Gen,
e) für Valin-Export-Proteine kodierende brnEF-Gene,
f) ein für eine Phosphoenolpyruvat-Carboxykinase kodierendes pck-Gen, und
g) ein für ein Malat-Enzym kodierendes mez-Gen, verstärkt vorliegt (vorliegen).

20. Verfahren nach Anspruch 16 oder 19, **dadurch gekennzeichnet, dass** in dem coryneformen Bakterium eines oder mehrere der Gene ausgewählt aus der Gruppe
a) ein für eine Pyruvat-Carboxylase kodierendes pyc-Gen,
b) ein für eine Pyruvat-Oxidase kodierends poxB-Gen,
c) ein für eine Phosphoenolpyruvat-Carboxylase kodierendes ppc-Gen,
d) ein für eine Threonin Deaminase kodierendes ilvA-Gen, und
e) ein für ein Valin-Importprotein kodierendes brnQ-Gen
abgeschwächt vorliegt (vorliegen).

21. Verfahren nach einem oder mehreren der Ansprüche 1 bis 11, **dadurch gekennzeichnet, dass** es sich bei der organisch-chemischen Verbindung um eine Ketosäure handelt.

22. Verfahren nach Anspruch 21, **dadurch gekennzeichnet, dass** es sich bei der Ketosäure um Brenztraubensäure oder Bernsteinsäure oder Ketoisovaleriansäure oder Ketoisocapronsäure handelt.

23. Verfahren nach einem oder mehreren der Ansprüche 1 - 22, **dadurch gekennzeichnet, dass** man die organisch-chemische Verbindung isoliert und reinigt.

24. Verfahren nach einem oder mehreren der Ansprüche 1 - 22, **dadurch gekennzeichnet, dass** Bestandteile der Fermentationsbrühe und/oder Biomasse in ihrer Gesamtheit oder in Anteilen (> 0 bis 100%) im Endprodukt verbleiben.

25. Verfahren nach einem oder mehreren der Ansprüche 1 - 22, **dadurch gekennzeichnet, dass** es sich um ein Satzverfahren handelt.

26. Verfahren nach einem oder mehreren der Ansprüche 1 - 22, **dadurch gekennzeichnet, dass** es sich um ein Zulaufverfahren handelt.

27. Verfahren nach einem oder mehreren der Ansprüche 1 - 22, **dadurch gekennzeichnet, dass** es sich um ein kontinuierliches Verfahren handelt.

28. Verfahren nach Anspruch 24, **dadurch gekennzeichnet, dass** man einer L-Lysin haltigen Fermentationsbrühe Wasser entzieht und ein Produkt mit einem Wassergehalt von maximal 5 Gew.-% erhält.

29. Verfahren nach Anspruch 24, **dadurch gekennzeichnet, dass** man eine L-Lysin haltige Fermentationsbrühe zunächst konzentriert und anschließend sprühtrocknet oder sprühgranuliert.

30. Verfahren nach Anspruch 29, **dadurch gekennzeichnet, dass** man die Oberfläche des erhaltenen Granulats mit einem Öl behandelt.

31. Coryneforme Bakterien, in denen das für die E1p Untereinheit des Pyruvat-Dehydrogenase Komplexes kodierende aceE-Gen abgeschwächt vorliegt.

32. Rekombinante coryneforme Bakterien, in denen das für die E1p Untereinheit des Pyruvat-Dehydrogenase Komplexes kodierende aceE-Gen abgeschaltet vorliegt.

33. Verfahren nach Anspruch 22, **dadurch gekennzeichnet, dass** es sich um Ketoisovaleriansäure handelt.

34. Verfahren nach Anspruch 33, **dadurch gekennzeichnet, dass** in dem coryneformen Bakterium eines oder mehrere der Gene ausgewählt aus der Gruppe
a) ein für eine Transaminase B kodierendes ilvE-Gen,
b) ein für eine Threonin Deaminase kodierendes ilvA-Gen,
c) ein für eine 2-Isopropylmalat Synthase kodierendes leuA-Gen,
d) ein für eine 3-Isopropylmalat Dehydrogenase kodierendes leuB-Gen,
e) ein für die grosse Untereinheit der Isopropylmalat Isomerase kodierendes leuC-Gen,
f) ein für die kleine Untereinheit der Isopropylmalat Isomerase kodierendes leuD-Gen,
g) ein für eine alpha-Ketoisovaleriansäure Hydroxymethyltransferase kodierendes panB-Gen, und
h) ein für eine Pantothenat Synthetase kodierendes panC-Gen
abgeschwächt vorliegt (vorliegen).

35. Verfahren nach Anspruch 33 und 34, **dadurch gekennzeichnet, dass** in dem coryneformen Bakterium eines oder mehrere der Gene ausgewählt aus der Gruppe
a) ein für eine IlvB-Untereinheit der Acetohydroxysäure Synthase kodieriendes ilvB-Gen,
b) ein für eine IlvN-Untereinheit der Acetohydroxysäure Synthase kodieriendes ilvN-Gen,
c) ein für eine Isomeroreduktase kodierendes ilvC-Gen,
d) ein für eine Dihydroxysäure Dehydratase kodierendes ilvD-Gen,
e) ein für eine Phosphoenolpyruvat-Carboxykinase kodierendes pckA-Gen, und
f) ein für ein Malat-Enzym (malic enzyme) kodierendes mez-Gen
überexprimiert vorliegt (vorliegen).

36. Verfahren nach Anspruch 22, **dadurch gekennzeichnet, dass** es sich um Ketoisocapronsäure handelt.

37. Verfahren nach Anspruch 36, **dadurch gekennzeichnet, dass** in dem coryneformen Bakterium eines oder mehrere der Gene ausgewählt aus der Gruppe
a) ein für eine IlvB-Untereinheit der Acetohydroxysäure Synthase kodieriendes ilvB-Gen,
b) ein für eine IlvN-Untereinheit der Acetohydroxysäure Synthase kodieriendes ilvN-Gen,
c) ein für eine Isomeroreduktase kodierendes ilvC-Gen,
d) ein für eine Dihydroxysäure Dehydratase kodierendes ilvD-Gen,
e) ein für eine 2-Isopropylmalat Synthase kodierendes leuA-Gen,
f) ein für eine 3-Isopropylmalat Dehydrogenase kodierendes leuB-Gen,
g) ein für die grosse Untereinheit der
Isopropylmalat Isomerase kodierendes leuC-Gen, und
h) ein für die kleine Untereinheit der
Isopropylmalat Isomerase kodierendes leuD-Gen überexprimiert vorliegt (vorliegen).

38. Verfahren nach Anspruch 36 und 37, **dadurch gekennzeichnet, dass** in dem coryneformen Bakterium eines oder mehrere der Gene ausgewählt aus der Gruppe
a) ein für eine alpha-Ketoisovaleriansäure Hydroxymethyltransferase kodierendes panB-Gen, und
b) ein für eine Pantothenat Synthetase kodierendes panC-Gen
abgeschwächt vorliegt (vorliegen).
